# EUROPÄISCHE PATENTSCHRIFT

(11) **EP 1 475 044 B1**
(45) Veröffentlichungstag und Bekanntmachung des Hinweises auf die Patenterteilung: **15.08.2007**
(21) Anmeldenummer: 04010112.3
(22) Anmeldetag: 28.04.2004
(51) Int. Cl.: A61B 17/02

(54) **Distraktionsvorrichtung**
Distraction device
Dispositif de distraction

(30) Priorität: 06.05.2003 EP 03010177; 22.12.2003 DE 10360433
(43) Veröffentlichungstag der Anmeldung: 10.11.2004
(73) Patentinhaber: Zimmer GmbH, 8404 Winterthur (CH)
(72) Erfinder: Stutz, Heinrich, 8500 Frauenfeld (CH); Dominati, Simon-Paul, 13007 Marseille (FR); Gyssler, Bernhard, 8800 Thalwil (CH); Houdemer, Hervé, 8400 Winterthur (CH); Romero, José, 8700 Küstnacht (CH); Castelli, Claudio, 24129 Bergamo (IT)
(74) Vertreter: Manitz, Finsterwald & Partner GbR

(56) Entgegenhaltungen:
- US-A- 4 501 266
- US-B1- 6 482 209

## Beschreibung

Die Erfindung betrifft eine hydraulische Distraktionsvorrichtung für Operationen, insbesondere für Knieoperationen gemäß dem Oberbegriff des Anspruchs 1. Eine derartige Vorrichtung ist aus der US-A-4 501 266 bekannt. Eine weitere Distraktionsvorrichtung ist in der US-B-6 482 209 offenbart.

Im Rahmen von Knieoperationen einsetzbare Distraktionseinrichtungen sind grundsätzlich bekannt und dienen dazu, das Knie aufzuspreizen, d.h. den Abstand zwischen Femur und Tibia zu vergrößern. Hiermit wird bezweckt, während der Knieoperation das Femur und die Tibia in ihre natürliche Relativlage zu bringen. Der Operateur versucht dabei, diese natürliche Relativlage anhand der natürlichen Spannung der Bänder zu ermitteln. Hierfür stehen dem Operateur keine zusätzlichen Hilfsmittel zur Verfügung, d.h. der Operateur legt die der weiteren Operation zugrunde liegende Relativlage zwischen Femur und Tibia ausschließlich "nach Gefühl" fest.

Auch bei anderen am Knie durchführenden Operationen ist der Operateur mangels zur Verfügung stehender zusätzlicher Hilfsmittel auf sein "Gefühl" angewiesen. So kommt es beispielsweise bei Implantationen von vorderen Kreuzbändern darauf an, dem Band beim Einsetzen eine Bandspannung zu verleihen, die dem natürlichen Bewegungsablauf des Kniegelenks entspricht.

Aufgabe der Erfindung ist es, eine Distraktionsvorrichtung für Operationen zu schaffen, die bei einfachem Aufbau und einfacher Handhabbarkeit die Möglichkeit bietet, dem Operateur während der Operation zusätzliche Informationen zur Verfügung zu stellen.

Die Lösung dieser Aufgabe erfolgt durch die Merkmale des Anspruchs 1 und insbesondere dadurch dass die Kolben-/Zylinder-Anordnung auswechselbar und aus Kunststoff hergestellt ist, wobei die Koppelung des Kolbens und des Zylinders mit den Arbeitsabschnitten jeweils lösbar ist und dass die Kolben-/Zylinder-Anordnung durch eine äußere, den Zylinder von außen abstützende Stützaufnahme sowie durch wenigstens einen inneren, zusammen mit dem Kolben in den Zylinder einführbaren Verstärkungseinsatz versteift ist.

Die Erfindung stellt eine hydraulische Distraktionsvorrichtung bereit, die sich dadurch auszeichnet, dass die Kolben-/Zylinder-Anordnung auswechselbar und aus Kunststoff hergestellt ist. Dies ermöglicht in vorteilhafter Weise die Verwendung kostengünstiger Wegwerfteile für die Kolben-/Zylinder-Anordnung, die folglich nicht wiederholt sterilisiert zu werden brauchen.

Die erfindungsgemäß vorgesehene Stützaufnahme und der zusammen mit dem Kolben in den Zylinder einführbare Verstärkungseinsatz erhöhen die Biegesteifigkeit der Kolben-/Zylinder-Anordnung. Hierdurch ist die Distraktionsvorrichtung trotz der Herstellung der eigentlichen Kolben-/Zylinder-Anordnung aus Kunststoff aufgrund der erfindungsgemäßen Versteifung insgesamt in der Lage, vergleichsweise große Kräfte aufzunehmen, so dass während der Operation auf die mit dem Kolben und dem Zylinder verbundenen Arbeitsabschnitte einwirkende äußere Kräfte von der Stützaufnahme und dem Verstärkungseinsatz aufgenommen werden können. Nicht akzeptable Verformungen der Kolben-/Zylinder-Anordnung werden hierdurch sicher vermieden, so dass zu jedem Zeitpunkt der Operation definierte geometrische Verhältnisse herrschen und eine insbesondere vorgesehene Parallelität von zungenförmigen Plattformen, die von den Arbeitsabschnitten gebildet werden, stets gegeben ist.

Bevorzugte Ausführungsformen der Erfindung sind in den Unteransprüchen angegeben.

So können der Kolben und der Zylinder jeweils im Spritzgussverfahren hergestellte Einwegteile sein. Sollte die Kolben-/Zylinder-Anordnung weitere Bestandteile umfassen, so sind vorzugsweise alle diese Bestandteile für eine Einmal-Verwendung vorgesehen, wodurch kostengünstige Herstellung und - aufgrund des Wegfalls der Notwendigkeit zu wiederholter Sterilisation - einfache Handhabung optimal kombiniert werden.

Vorzugsweise sind die Stützaufnahme und der Verstärkungseinsatz aus einem Material mit einer höheren Biegesteifigkeit als der Kunststoff der Kolben-/Zylinder-Anordnung hergestellt. Insbesondere wird hierzu Metall oder faserverstärkter Kunststoff verwendet.

Der Kolben und der Verstärkungseinsatz können im zusammengesetzten Zustand eine kompakte Kolbeneinheit bilden, die zumindest über einen Teilumfangsbereich das Innere des Zylinders vollständig ausfüllt.

In einem besonders bevorzugten Ausführungsbeispiel ist die Kolben-/Zylinder-Anordnung von einer handelsüblichen Einwegspritze gebildet. Hierdurch wird auf besonders elegante Weise ein äußerst kostengünstig herstellbarer Massenartikel zu einem zentralen Bestandteil einer hydraulischen Distraktionsvorrichtung für Knieoperationen.

Ferner kann erfindungsgemäß vorgesehen sein, dass ein zwischen der Innenwand des Zylinders und einer Kolbenstange vorhandener Freiraum durch den Verstärkungseinsatz zumindest teilweise ausgefüllt ist. Hierdurch können ohnehin vorhandene Freiräume, wie sie handelsübliche Einwegspritzen aufweisen, für eine versteifende Verstärkung oder Hinterfütterung mittels des wenigstens einen Verstärkungseinsatzes genutzt werden.

Des Weiteren kann wenigstens eine im Zylinder vorhandene Kammer, die durch eine bis an die Innenwand des Zylinders reichende, insbesondere im Querschnitt kreuz- oder X-förmige Rippenstruktur einer Kolbenstange begrenzt ist, durch den Verstärkungseinsatz zumindest teilweise ausgefüllt sein.

Dabei kann wenigstens ein Paar einander gegenüberliegender Kammern jeweils durch einen Verstärkungseinsatz zumindest teilweise ausgefüllt sein.

Des Weiteren wird erfindungsgemäß vorgeschlagen, dass der Verstärkungseinsatz das Innere einer rohrförmigen, an der Innenwand des Zylinders geführten Kolbenstange zumindest über einen Teilumfangsbereich vollständig ausfüllt.

Es ist erfindungsgemäß nicht zwangsläufig erforderlich, den Kolben derart zu verstärken, dass der Zylinder über seine gesamte Querschnittsfläche vollständig ausgefüllt ist. Bevorzugt ist es, wenn sich die innere Verstärkung über die gesamte zur Verfügung stehende Länge der Kolben-/Zylinder-Anordnung erstreckt. Hierdurch ist die Stabilität und Biegesteifigkeit der Kolben-/Zylinder-Anordnung für jede Kolbenstellung und insbesondere auch bei vergleichsweise weit ausgefahrenem Kolben gewährleistet.

Die Kolben-/Zylinder-Anordnung kann derart ausgebildet sein, dass sie im zusammengesetzten Zustand mit den Arbeitsabschnitten gekoppelt werden kann. Hierdurch wird die Handhabbarkeit der erfindungsgemäßen Distraktionsvorrichtung erheblich erleichtert.

Wenigstens ein Verstärkungseinsatz kann fest mit dem Arbeitsabschnitt verbunden sein. Dies ermöglicht eine besonders stabile Konstruktion von hoher Steifigkeit.

Des Weiteren kann wenigstens ein Verstärkungseinsatz in Form eines separaten Bauteils vorgesehen sein.

Die Stützaufnahme für den Zylinder und der mit dem Zylinder koppelbare Arbeitsabschnitt können fest mit einem Tragkörper verbunden sein. Die Anzahl einzelner Bauteile der erfindungsgemäßen Distraktionsvorrichtung wird hierdurch besonders gering gehalten, was die Handhabung und insbesondere die Sterilisation der Vorrichtung in vorteilhafter Weise erleichtert.

Auf der den Arbeitsabschnitten gegenüberliegenden Seite der Kolben-/Zylinder-Anordnung kann ein Handgriff vorgesehen sein, der bevorzugt fest mit dem Tragkörper verbunden ist.

Ferner wird erfindungsgemäß vorgeschlagen, dass der mit dem Zylinder koppelbare Arbeitsabschnitt fest mit einem sich parallel zur Distraktionsrichtung erstreckenden Funktionsblock verbunden ist, der als Verdreh- und/oder Auslenksicherung für den mit dem Kolben koppelbaren Arbeitsabschnitt ausgebildet ist.

Durch einen derartigen Funktionsblock kann für alle Kolbenstellungen eine korrekte Relativlage zwischen den beiden Arbeitsabschnitten auch bei relativ großen auf die Arbeitsabschnitte einwirkenden äußeren Kräften sichergestellt werden.

Der Funktionsblock kann einen von einer Kreisform abweichenden Außenquerschnitt aufweisen und zumindest über einen Teilumfangsbereich von einem Führungsabschnitt des Arbeitsabschnitts verdrehsichernd umgriffen sein.

Ferner kann vorgesehen sein, dass der Funktionsblock eine geringfügig gegenüber der Distraktionsrichtung geneigte, in Richtung zunehmender Distraktionslänge auf die Längsachse der Kolben-/Zylinder-Anordnung zu laufende Anschlagfläche aufweist, die mit dem Arbeitsabschnitt auslenkungssichernd zusammenwirkt.

Vorzugsweise ist der Funktionsblock mit einer Anzeigeeinrichtung versehen, an der ein Maß für die Distraktionslänge ablesbar ist. Die Anzeigeeinrichtung kann in Form einer an einer Außenseite des Funktionsblocks angebrachten Skala vorgesehen sein. Indem auf diese Weise ein Maß für die Distraktionslänge und damit für den Abstand zwischen den distrahierten Arbeitsabschnitten zur Verfügung steht, können Knieoperationen auf einfache und zuverlässige Weise reproduzierbar gestaltet werden.

Des Weiteren kann die Kolben-/Zylinder-Anordnung mit einer Anzeigeeinrichtung, insbesondere in Form einer am Zylinder angebrachten Skala, versehen sein. Im Fall der Verwendung einer Einwegspritze als Kolben-/Zylinder-Anordnung kann die ohnehin am Zylinder der Spritze angebrachte Skala genutzt werden.

Des Weiteren wird erfindungsgemäß vorgeschlagen, dass eine Fluidzuführeinrichtung für die Kolben-/Zylinder-Anordnung eine von Hand betätigbare Spritze umfasst, insbesondere eine Einwegspritze.

An eine die Kolben-/Zylinder-Anordnung mit einer Fluidzuführeinrichtung verbindende Fluidleitung kann wenigstens eine Druckanzeigeeinrichtung angeschlossen sein. Hierdurch steht ein Maß für die zwischen den Arbeitsabschnitten wirksame Distraktionskraft zur Verfügung. Werden bei der Operation Bänder gespannt, so kann folglich an der Druckanzeigeeinrichtung ein Maß für die jeweilige Bandspannung abgelesen werden. Dies ermöglicht es wiederum, die Operationen reproduzierbar zu gestalten.

In einer weiteren bevorzugten Ausführungsform der Erfindung ist die Kolben-/Zylinder-Anordnung ein Bestandteil eines geschlossenen, als eine Einheit handhabbaren Hydrauliksystems, das zusätzlich zumindest eine Fluidzuführeinrichtung, eine die Kolben-/Zylinder-Anordnung mit der Fluidzuführeinrichtung verbindende Fluidleitung sowie ein Hydraulikfluid, insbesondere Wasser, umfasst und als Ganzes mit den Arbeitsabschnitten koppelbar ist. Ein solches geschlossenes Hydrauliksystem kann insbesondere als Ganzes sterilisiert und mit den übrigen Bauteilen oder Baugruppen der Distraktionsvorrichtung gekoppelt werden, was die Benutzung erheblich erleichtert. Bei der Vorbereitung oder während der Operation braucht das vorher sterilisierte Hydrauliksystem lediglich der Verpackung entnommen und mit den Arbeitsabschnitten gekoppelt zu werden. Umständliche und zeitraubende Anschluss- oder Entlüftungsarbeiten sind nicht erforderlich.

Gemäß einem weiteren Ausführungsbeispiel der Erfindung sind wenigstens zwei im mit den Arbeitsabschnitten gekoppelten Zustand in parallelen Richtungen wirksame Kolben-/Zylinder-Anordnungen vorgesehen.

Eine Vorrichtung mit zwei nebeneinander liegenden Kolben-/Zylinder-Anordnungen jeweils mit zugehörigen Arbeitsabschnitten kann insbesondere für bikompartimentale Operationen zum Einsetzen von bikondylären Knieprothesen verwendet werden.

Bevorzugt sind die Kolben-/Zylinder-Anordnungen unabhängig voneinander ansteuerbar, d.h. mit Hydraulikfluid beaufschlagbar, so dass der Abstand zwischen Femur und Tibia in den beiden Kompartimenten unterschiedlich groß eingestellt werden kann.

Bevorzugt sind die Kolben-/Zylinder-Anordnungen während der Benutzung an eine gemeinsame Fluidzuführeinrichtung angeschlossen.

Ferner können durch in einer die Kolben-/Zylinder-Anordnungen mit einer Fluidzuführeinrichtung verbindenden Fluidleitung angeordnete Schaltventile die Kolben-/Zylinder-Anordnungen unabhängig voneinander jeweils wahlweise mit der Fluidzufuhr verbindbar oder von der Fluidzufuhr trennbar sein.

Zum Einstellen von Sollabständen zwischen Femur und Tibia sind die Arbeitsabschnitte bevorzugt derart ausgebildet, dass sie einen zwischen Femur und Tibia einbringbaren und sich im Wesentlichen senkrecht zur Distraktionsrichtung erstreckenden Aufspreizabschnitt bilden. Die Arbeitsabschnitte können jeweils in Form einer plattenförmigen Zunge vorgesehen sein.

Die erfindungsgemäße Distraktionsvorrichtung ist nicht ausschließlich zum Auseinanderdrücken von Tibia und Femur verwendbar, sondern kann auch im Rahmen anderer Operationen insbesondere im Bereich des Knies verwendet werden, bei denen Teile relativ zueinander bewegt werden sollen und es wünschenswert ist, während des Operationsverlaufs zusätzliche Informationen insbesondere über die momentanen Werte der Distraktionslänge der Distraktonskraft zu erhalten.

So kann die erfindungsgemäße Distraktionsvorrichtung beispielsweise bei der Implantation vorderer Kreuzbänder dazu verwendet werden, das am Femur fixierte Band zu spannen, um die Fixierung des Bandes an der Tibia bei einer für die natürliche Bewegung des Kniegelenks passenden Bandspannung durchzuführen.

Zur Bestimmung der Bandspannung und/oder Bandlänge bei der Implantation von vorderen Kreuzbändern kann ein oberer Arbeitsabschnitt mit einem Abstütz- und/oder Befestigungsabschnitt versehen sein, über den die Vorrichtung an der Tibia abstützbar und/oder befestigbar ist.

Die Arbeitsabschnitte können jeweils mit wenigstens einer Aussparung zum Hindurchführen eines Fixierwerkzeugs und eines Fixierelementes zum Fixieren des Bandes in der Tibia versehen sein. Bei dem Werkzeug handelt es sich beispielsweise um einen Schraubendreher, mit dem eine das Fixierelement bildende Verdrängerschraube in eine in der Tibia ausgebildete Bohrung eingeschraubt werden kann, um das zu implantierende, in die Bohrung eingeführte Band festzusetzen. Die in den Arbeitsabschnitten ausgebildete Aussparung ist vorzugsweise derart bemessen, dass der Schraubendreher mit aufgesetzter Verdrängerschraube entlang dem Band eingebracht werden kann.

Des Weiteren wird vorgeschlagen, dass der obere Arbeitsabschnitt mit wenigstens einer Aussparung zum Hindurchführen des Bandes und/oder eines mit dem Band verbundenen Zugelementes versehen ist.

Ferner kann ein unterer Arbeitsabschnitt mit wenigstens einem Befestigungsabschnitt zum Befestigen des Bandes oder eines mit dem Band verbundenen Zugelementes versehen sein.

Gemäß einem weiteren bevorzugten Ausführungsbeispiel der Erfindung ist vorgesehen, dass die Stützaufnahme für den Zylinder zumindest teilrohrförmig ausgebildet und der Zylinder längs der Rohrachse in die Stützaufnahme einführbar ist, wobei die Stützaufnahme zum seitlichen Einlegen einer mit dem Zylinder verbundenen Fluidleitung eine insbesondere schlitzförmige Einlegeöffnung aufweist.

Hierdurch ist es beim Vorbereiten der Distraktionsvorrichtung in vorteilhafter Weise möglich, dass der Zylinder - wenn er mit demjenigen Ende, mit welchem er an die Fluidleitung angeschlossen ist, voran in Stützaufnahme eingeführt werden soll - bereits mit der Fluidleitung verbunden sein kann, so dass es zu keinem Zeitpunkt während des Zusammensetzens der Distraktionsvorrichtung erforderlich ist, die Fluidverbindung zwischen Fluidleitung und Zylinder zu trennen.

Des Weiteren wird gemäß einem bevorzugten Ausführungsbeispiel vorgeschlagen, dass ein Träger für eine Schnittlehre, insbesondere in Form eines Schnittblocks, vorgesehen ist, der mit einem Tragkörper koppelbar ist, mit dem die Stützaufnahme für den Zylinder und der mit dem Zylinder koppelbare Arbeitsabschnitt verbunden sind.

Mittels eines derartigen Trägers kann ein Schnittblock zum Vorbereiten des Femurs bzw. der Femurkondylen in eine beliebig einstellbare Höhe relativ zum Tragkörper und damit zu den Arbeitsabschnitten der Distraktionsvorrichtung gebracht werden.

Vorzugsweise umfasst der Träger eine als Auflage für die Schnittlehre dienende Plattform und eine mit dem Tragkörper koppelbare Tragstange, wobei die Tragstange an ihrem einen Ende die Plattform trägt und in einen Verstellkanal des Tragkörpers einführbar ist, in welchem die Tragstange relativ zum Tragkörper verstellbar und in einer Vielzahl von insbesondere diskret verteilten Positionen am Tragkörper fixierbar ist.

Dabei ist bevorzugt vorgesehen, dass die Tragstange und die Innenwand des Verstellkanals mit zur Fixierung ihrer Relativlage zusammenwirkenden, insbesondere rippenförmigen Profilierungen versehen sind.

Bevorzugt ist die Verstellanordnung aus Tragstange und Verstellkanal selbsthemmend ausgebildet.

Die Tragstange kann zumindest bereichsweise gabelförmig ausgebildet sein und gegen eine Rückstellkraft zusammendrückbare Gabelarme umfassen, die insbesondere auf ihren einander abgewandten Außenseiten mit Profilierungen versehen sind. Hierdurch kann einfach durch Zusammendrücken der beiden Gabelarme die Tragstange vom Tragkörper gelöst und im Verstellkanal bewegt werden, um in eine neue Lage relativ zum Tragkörper gebracht zu werden.

Die Gabelarme der Tragstange können insbesondere an in entgegengesetzte Richtungen weisenden Seiten mit Profilierungen in Form von unterschiedlich orientierten Rippenstrukturen versehen sein.

In einem weiteren bevorzugten Ausführungsbeispiel ist vorgesehen, dass die Tragstange und der Tragkörper zumindest bei Verstellbewegungen längs der Distraktionsachse nach Art einer Ratsche zusammenwirken. In einer Richtung, z.B. zum Anheben der Plattform, braucht somit die Tragstange lediglich längs des Verstellkanals bewegt zu werden, ohne die Gabelarme zusammenzudrücken.

Ferner kann gemäß einem weiteren Ausführungsbeispiel die Tragstange aus einer parallelen Ausrichtung zur Distraktionsachse heraus gegenüber dem Tragkörper verschwenkbar ist, insbesondere in einer von zwei zu beiden Seiten des Verstellkanals angeordneten Kolben-/Zylinder-Anordnungen festgelegten Ebene. Hierdurch kann die Plattform und damit ein auf der Plattform aufliegender Schnittblock bezüglich der Distraktionsachse schräg gestellt werden.

Die Tragstange kann einen auf die Breite des Verstellkanals abgestimmten Schwenkkopf aufweisen, um welchen die Tragstange verschwenkbar ist.

Des Weiteren kann sich der Verstellkanal in bei eingesteckter Tragstange von der Plattform weg weisender Richtung erweitern.

Die Tragstange kann mit einer Anzeigeeinrichtung, insbesondere in Form einer Skala, versehen sein, die im in den Verstellkanal eingeführten Zustand über ein in einer Wand des Tragkörpers ausgebildetes Ablesefenster von außen sichtbar ist. Ferner kann der obere Arbeitsabschnitt mit einer Anzeigeeinrichtung, insbesondere in Form einer Skala, versehen sein. Auf diese Weise können das Arbeitsniveau der Plattform sowie des oberen Arbeitsabschnitts in Bezug auf den Tragkörper abgelesen werden, woraus sich die Höhenlage der Plattform und damit eines auf der Plattform aufliegenden Schnittblocks in Bezug auf den oberen Arbeitsabschnitt bestimmen lässt.

Des Weiteren ist erfindungsgemäß vorgesehen, dass die Plattform bei zum Aufspreizen des Knies zwischen Femur und Tibia eingebrachten Arbeitsabschnitten zumindest im Wesentlichen außerhalb der Projektion der Tibia längs der Distraktionsachse gelegen ist. Ferner kann die Plattform vor einem Funktionsblock gelegen sein, der sich parallel zur Distraktionsachse erstreckt und mit dem der mit dem Zylinder koppelbare Arbeitsabschnitt fest verbunden ist.

Durch eine derartige Anordnung der Plattform lässt sich eine besonders einfache und problemlose Handhabung der Distraktionsvorrichtung realisieren.

Die Erfindung wird im Folgenden beispielhaft unter Bezugnahme auf die Zeichnung beschrieben. Es zeigen:
- Fig. 1: einen Längsschnitt durch eine Distraktionsvorrichtung gemäß einer Ausführungsform der Erfindung,
- Fig. 2: eine Vorderansicht der Distraktionsvorrichtung von Fig. 1,
- Fig. 3: eine Draufsicht auf die Distraktionsvorrichtung von Fig. 1 ohne Zylinder und ohne obere Arbeitsabschnitte,
- Fig. 4: eine Draufsicht entsprechend Fig. 3 mit eingeführten Zylindern und oberen Arbeitsabschnitten,
- Fig. 5: eine Möglichkeit für den Anschluss einer erfindungsgemäßen Distraktionsvorrichtung an eine Fluidzuführeinrichtung,
- Fig. 6: eine Möglichkeit für den Anschluss einer erfindungsgemäßen Distraktionsvorrichtung an zwei unabhängig voneinander betreibbare Fluidzuführeinrichtungen,
- Fig. 7: einen Teillängsschnitt durch eine Distraktionsvorrichtung gemäß einer weiteren Ausführungsform der Erfindung,
- Fig. 8: die Distraktionsvorrichtung von Fig. 7 als Bestandteil eines geschlossenen Hydrauliksystems,
- Fig. 9 - 11: die Verwendung erfindungsgemäßer Distraktionsvorrichtungen bei der Implantation eines vorderen Kreuzbandes,
- Fig. 12: eine perspektivische Ansicht einer Distraktionsvorrichtung gemäß einer weiteren Ausführungsform der Erfindung,
- Fig. 13 - 15: verschiedene Ansichten der Distraktionsvorrichtung von Fig. 12,
- Fig. 16 - 19: verschiedene Ansichten eines Tragkörpers der Distraktionsvorrichtung von Fig. 12,
- Fig. 20 und 21: verschiedene Ansichten eines Trägers der Distraktionsvorrichtung von Fig. 12,
- Fig. 22: verschiedene Ansichten einer Tragstange des Trägers der Fig. 20 und 21, und
- Fig. 23 und 24: jeweils eine perspektivische Ansicht eines linken bzw. rechten oberen Arbeitsabschnittes mit zugehörigen Verstärkungseinsätzen.

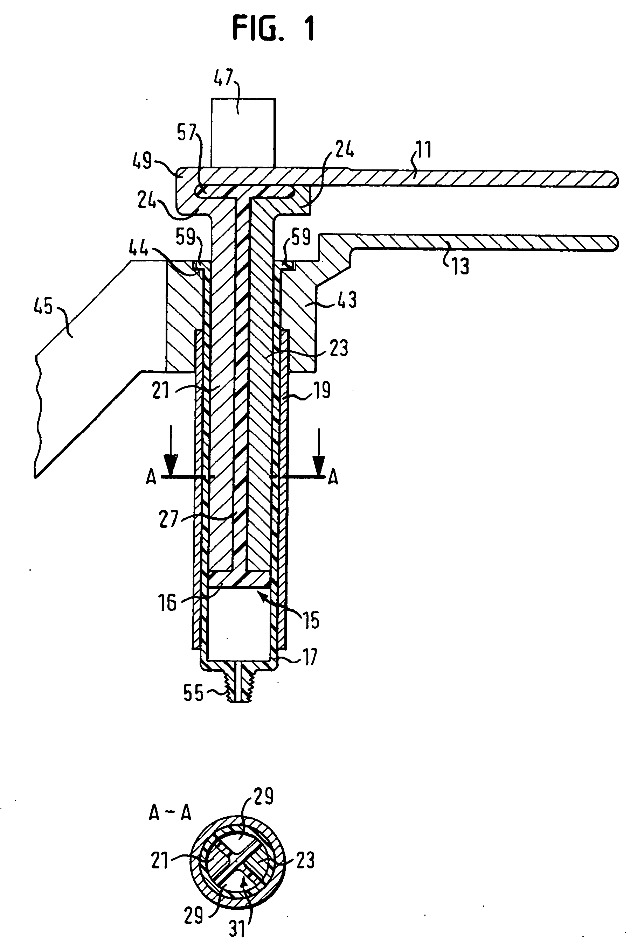

Die in den Fig. 1 - 4 dargestellte Ausführungsform einer erfindungsgemäßen hydraulischen Distraktionsvorrichtung umfasst einen Tragkörper 43, der auf einer Seite einen Handgriff 45 trägt und auf der gegenüberliegenden Seite einteilig mit einer zungenförmigen Plattform verbunden ist, die einen unteren Arbeitsabschnitt 13 der Distraktionsvorrichtung bildet.

Im Tragkörper 43 sind ferner zwei parallel verlaufende Durchgänge ausgebildet, die jeweils nach unten in ein am Tragkörper 43 befestigtes Stützrohr 19 übergehen. Auf der von den Stützrohren 19 abgewandten Seite weist der Tragkörper 43 einen mittig zwischen den Durchgängen angeordneten Funktionsblock 47 auf, auf den an anderer Stelle näher eingegangen wird.

In die Durchgänge des Tragkörpers 43 eingeführt ist jeweils ein Zylinder 17 einer in diesem Ausführungsbeispiel in Form einer handelsüblichen Einwegspritze vorgesehenen Kolben-/Zylinder-Anordnung. In ihrer Sollstellung stützen sich die Zylinder 17 jeweils mit Laschen 59 an einer entsprechend ausgebildeten Ringschulter 44 des Tragkörpers 43 ab. Die Zylinder 17 liegen jeweils mit ihrer Außenwand an der Innenwand des Stützrohres 19 an. Auf jeweils am freien Ende der Zylinder 17 ausgebildete Anschlussabschnitte 55 sind in den Fig. 1 - 4 nicht dargestellte Fluidleitungen aufschraubbar.

Jede Einwegspritze umfasst des Weiteren einen Kolben 15 mit einem an der Innenwand des Zylinders 17 fluiddicht anliegenden Kolbenabschnitt 16 und einer Kolbenstange 27, die an ihrem vom Kolbenabschnitt 16 abgewandten freien Ende in einen auch als Pilz bezeichneten, radial gegenüber der Kolbenstange 27 erweiterten Kopfabschnitt 57 übergeht.

Die Führung des Kolbens 15 im Bereich der Kolbenstange 27 erfolgt durch eine kreuzförmige Rippenstruktur 31 (vgl. die Querschnittsansicht A-A unten in Fig. 1). Die Kolbenstange 27 umfasst vier Rippen, wobei jeweils zwei benachbarte Rippen einen Winkel von 90° einschließen. Bei in den Zylinder 17 eingeschobenem Kolben 15 begrenzen die Rippen der Kolbenstange 27 Kammern 29 innerhalb des Zylinders 17. In dem dargestellten Ausführungsbeispiel sind zwei einander gegenüberliegende Kammern (vgl. die Querschnittsansicht A-A) jeweils vollständig durch einen Verstärkungseinsatz 21 bzw. 23 ausgefüllt.

Der eine Verstärkungseinsatz 21 ist einstückig mit einer weiteren zungenförmigen Plattform verbunden, die den oberen Arbeitsabschnitt 11 der erfindungsgemäßen Distraktionsvorrichtung bildet. Der andere Verstärkungseinsatz 23 ist in Form eines separaten Bauteils vorgesehen. Der mit dem oberen Arbeitsabschnitt 11 verbundene Einsatz 21, der separate Einsatz 23 sowie der Kolben 15 werden zunächst zu einer Kolbeneinheit zusammengesetzt, bevor diese als Ganzes in den Zylinder 17 eingeschoben wird, wobei der Zylinder 17 zuvor bereits in den entsprechenden Durchgang des Tragkörpers 43 eingeschoben worden sein kann.

Beim Zusammensetzen der Kolbeneinheit wird der pilzförmige Kopf 57 der Kolbenstange 27 zunächst mit einer Seite in eine schlitzförmige Aufnahme zwischen dem oberen Arbeitsabschnitt 11 und einem sich parallel zu dem oberen Arbeitsabschnitt 11 erstreckenden Flanschabschnitt 24 eingeführt, der Bestandteil eines als Führungsabschnitt 49 dienenden Übergangsabschnitts zwischen dem oberen Arbeitsabschnitt 11 und dem mit diesem verbundenen Verstärkungseinsatz 21 ist.

Der separate Verstärkungseinsatz 23 ist mit einem entsprechenden Flanschabschnitt 24 versehen, so dass der Kopf 57 der Kolbenstange 27 zwischen den beiden Einsätzen 21, 23 und dem oberen Arbeitsabschnitt 11 räumlich fixiert ist.

Beim Einführen des Kopfes 57 in den im Führungsabschnitt 49 ausgebildeten Aufnahmeschlitz gelangt der mit dem oberen Arbeitsabschnitt 11 verbundene Einsatz 21 in die entsprechende Kammer (vgl. Querschnittsansicht A-A in Fig. 1) der Rippenstruktur 31. Entsprechendes gilt beim Einfügen des separaten Einsatzes 23.

Mit den beiden Einsätzen 21, 23 werden folglich für jede Stellung des Kolbens 15 zwei einander gegenüberliegende Kammern innerhalb des Zylinders 17 vollständig mit Material ausgefüllt, wodurch der Kolbeneinheit eine hohe Biegesteifigkeit verliehen wird. Durch das Stützrohr 19 wird der Zylinder 17 ferner von außen abgestützt, so dass insgesamt eine biegesteife Kolben-/Zylinder-Anordnung geschaffen wird, die auch bei großen auf die beiden Arbeitsabschnitte 11, 13 einwirkenden äußeren Kräften stets eine ausreichende Parallelität zwischen den beiden Arbeitsabschnitten 11, 13 sicherstellt. Dabei dient in vorteilhafter Weise das obere freie Ende des separaten Einsatzes 23 im Bereich des Flanschabschnitts 24 als Abstützung für den oberen Arbeitsabschnitt 11.

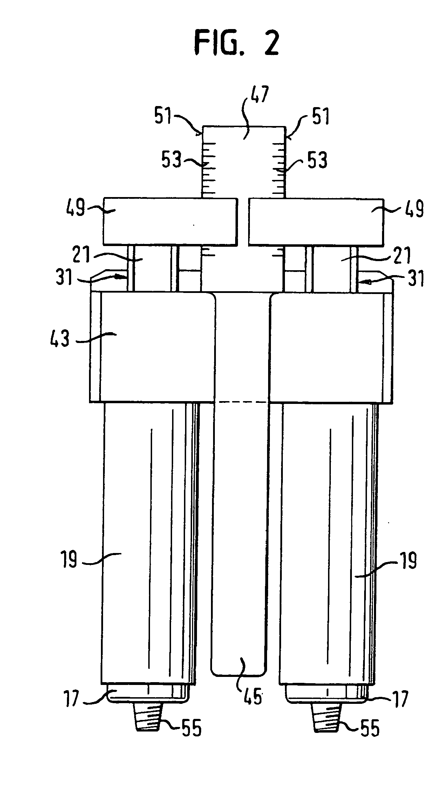

Die Vorderansicht der Fig. 2 zeigt insbesondere den mittig bezüglich der Stützrohre 19, aus denen die Zylinder 17 unten jeweils ein Stück weit heraus ragen, angeordneten Handgriff 45. Außerdem in Fig. 2 zu erkennen sind die in Richtung des Handgriffs 45 weisenden Seiten der fest mit dem oberen Arbeitsabschnitt 11 verbundenen Einsätze 21 sowie jeweils der beiden die Kammer für den Einsatz 21 begrenzenden Rippen der Rippenstruktur 31.

Der Funktionsblock 47 ist auf seiner in Richtung des Handgriffs 45 weisenden Seite mit Skalen 53 versehen, an denen die jeweilige Kolbenstellung abgelesen werden kann, wodurch ein Maß für die Distraktionslänge zur Verfügung steht.

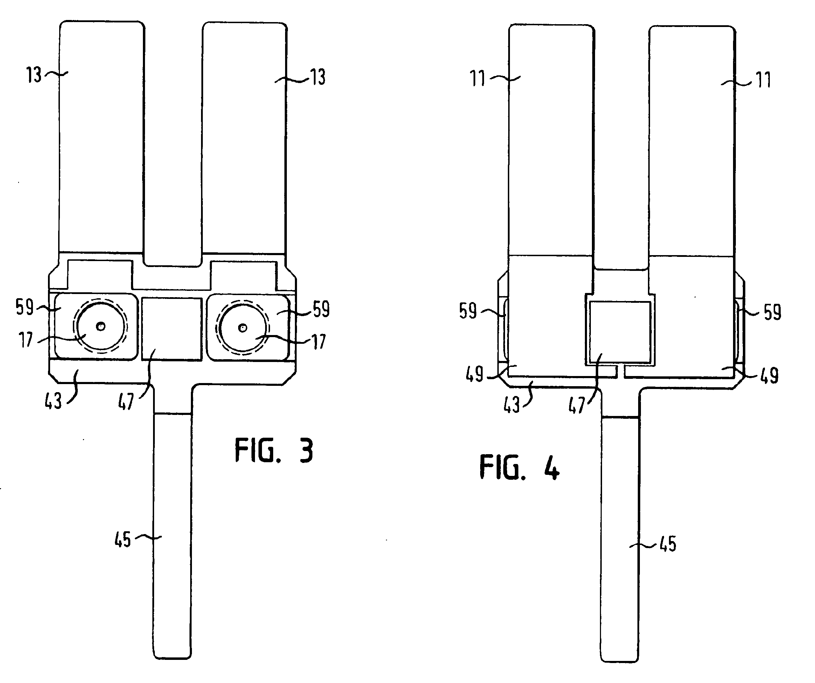

Wie insbesondere aus Fig. 4 hervorgeht, wird der Funktionsblock 47 von den Führungsabschnitten 49 jeweils in einen Eckbereich umgriffen und dient somit als eine Führung für die jeweils aus Kolbeneinheit und oberem Arbeitsabschnitt 11 bestehenden Baugruppen. Insofern stellen die Führungsabschnitte 49 jeweils einen Führungsabschnitt für die betreffende Baugruppe dar, der ein Verdrehen der Baugruppe relativ zu dem Tragkörper 43 um die Längsachse der Kolben-/Zylinder-Einheit sicher verhindert und somit eine gleichbleibende Orientierung des jeweiligen oberen Arbeitsabschnitts 11 sicherstellt.

Abweichend von der dargestellten Ausführungsform kann der Funktionsblock 47 derart trapezförmig ausgebildet sein, dass er sich nach oben verbreitert, so dass - anders als in Fig. 2 - die Außenseiten 53 nicht parallel, sondern geneigt zur Distraktionsrichtung verlaufen. Ein bei vollständig eingeschobener Kolbeneinheit noch vorhandenes, geringes Spiel zwischen Führungsabschnitt 49 und Funktionsblock 47 wird auf diese Weise mit zunehmender Distraktionslänge reduziert.

Fig. 3, in der die erfindungsgemäße Distraktionsvorrichtung ohne in die Zylinder 17 eingeschobene Kolbeneinheiten und damit ohne obere Arbeitsabschnitte 11 dargestellt ist, zeigt insbesondere die bezüglich der Längsachse rechtwinklig abstehenden Laschen 59, mit denen die Zylinder 17 jeweils auf den hierfür vorgesehenen Ringschultern 44 des Tragkörpers 43 aufliegen.

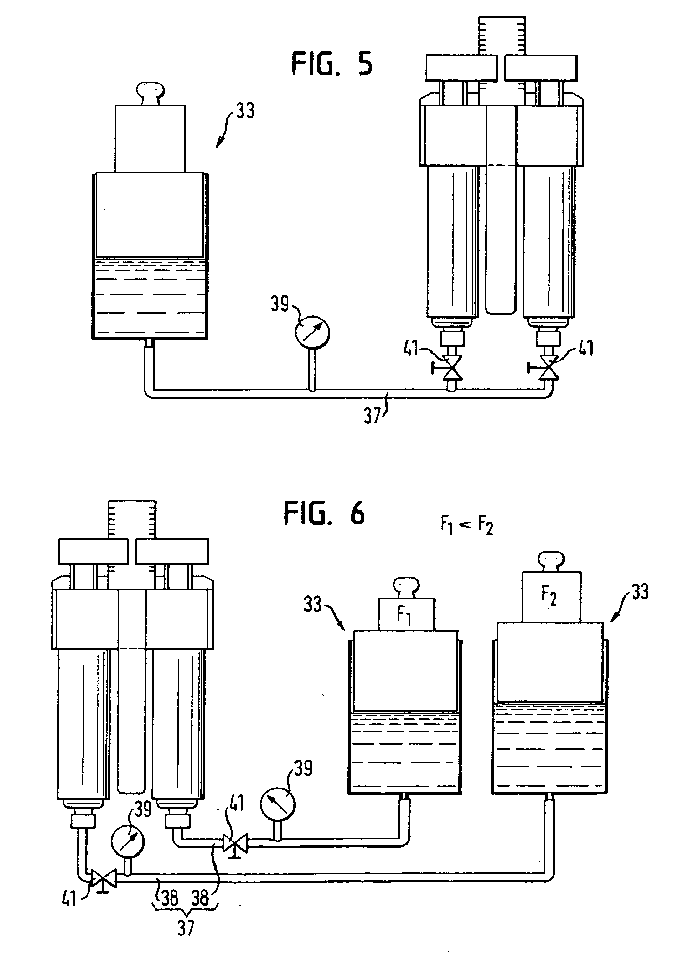

Wie Fig. 5 zeigt, können beide Kolben-/Zylinder-Anordnungen an eine gemeinsame, hier nur prinzipiell dargestellte Fluidzuführeinrichtung 33 angeschlossen werden. Schaltventile 41 ermöglichen es, die beiden Einheiten insofern unabhängig voneinander zu betreiben, als die eine Einheit durch Schließen des betreffenden Ventils 41 bei der gerade erreichten Distraktionslänge festgesetzt und bei der anderen Einheit durch Offenlassen des Ventils 41 die Distraktion fortgesetzt wird. Hierdurch kann beispielsweise beim Aufspreizen eines Knies mit einer asymmetrischen Kräfteverteilung gearbeitet werden.

An einer an die gemeinsame Fluidleitung 37 angeschlossenen Druckanzeigeeinrichtung 39 kann ein Maß für die zwischen den Arbeitsabschnitten wirksame Distraktionskraft abgelesen werden

Fig. 6 zeigt ein Beispiel, bei dem die beiden Kolben-/Zylinder-Anordnungen der Distraktionsvorrichtung vollkommen unabhängig voneinander betrieben werden können. Jeder Einheit ist eine eigene Fluidzuführeinrichtung 33 zugeordnet, die über eine Einzelleitung 38 an den jeweiligen Zylinder angeschlossen ist. Jeder Einzelleitung 38 ist ein Schaltventil 41 zum wahlweisen Absperren oder Freigeben der Einzelleitung 38 sowie eine Druckanzeigeeinrichtung 39 zugeordnet.

Bei den in Fig. 5 und 6 dargestellten Fluidzuführeinrichtungen 33 kann es sich um handelsübliche Einwegspritzen 33 handeln. Diese können von gleicher Bauart sein wie die die Kolben-/Zylinder-Anordnungen 15, 17 bildenden Einwegspritzen, sofern das insgesamt für die beiden Kolben- / Zylinder-Anordnungen 15, 17 benötigte Fluidvolumen mit einer einzigen Einwegspritze zur Verfügung gestellt werden kann. Zumindest für den überwiegenden Teil von unter Zuhilfenahme der erfindungsgemäßen Distraktionsvorrichtung durchführbaren Operationen ist dies der Fall, da man in den meisten Fällen ohnehin bestrebt ist, ein übermäßiges Ausfahren der Kolbeneinheiten zu vermeiden.

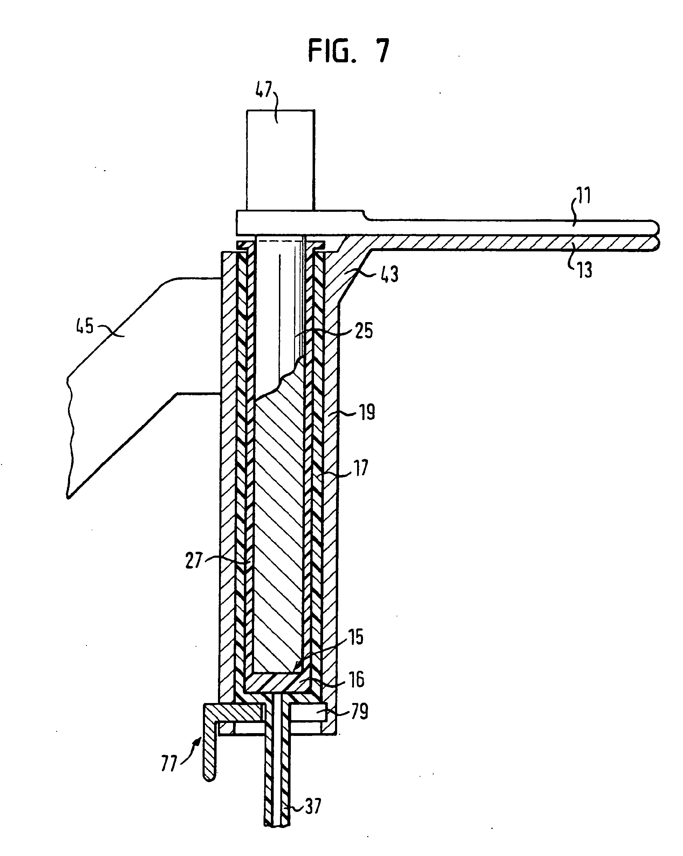

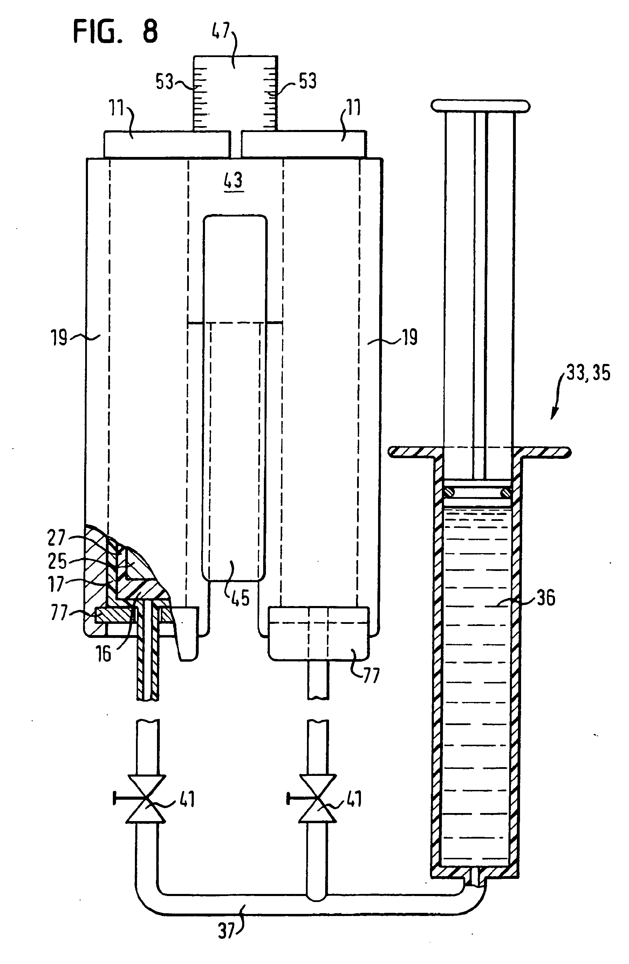

In dem weiteren Ausführungsbeispiel einer erfindungsgemäßen Distraktionsvorrichtung gemäß Fig. 7 und 8 ist lediglich ein einziger Verstärkungseinsatz 25 vorgesehen, der fest und insbesondere einteilig mit dem oberen Arbeitsabschnitt 11 ausgebildet ist. Der Einsatz 25 ist ein Vollzylinder, der mit seiner Außenwand an der Innenwand einer in diesem Ausführungsbeispiel rohrförmigen Kolbenstange 27 anliegt, so dass das Innere der Kolbenstange 27 durch den Einsatz 25 vollständig ausgefüllt ist.

Der einteilig mit einer in Fig. 7 nur teilweise dargestellten Fluidleitung 37 verbundene Zylinder 17 der Kolben-/Zylinder-Anordnung wird in dieser Ausführungsform von unten in das mit dem Tragkörper 43 verbundene Stützrohr 19 eingeführt. Eine Verriegelungseinrichtung 77 mit einem gabelförmigen Verriegelungsabschnitt 79 dient zur Sicherung des eingeschobenen Zylinders 17. Aus nachstehend erläuterten Gründen erfolgt das Einschieben des Zylinders 17 in das Stützrohr 19 bei in den Zylinder 17 eingeführtem Kolben 15, so dass zur Komplettierung der Distraktionsvorrichtung anschließend nur noch die vollzylindrischen Einsätze 25 in die rohrförmigen Kolbenstangen 27 eingeschoben zu werden brauchen.

Wie Fig. 8 zeigt, sind die beiden Kolben-/Zylinder-Anordnungen Bestandteile eines geschlossenen Hydrauliksystems, das außerdem eine gemeinsame Fluidzuführeinrichtung 33 in Form einer Einwegspritze 35, ein Hydraulikfluid 36, insbesondere Wasser, sowie eine die beiden Zylinder mit der Spritze 33 verbindende Fluidleitung 37 mit Schaltventilen 41 jeweils in Form eines Absperrhahns umfasst. Zusätzlich kann die Fluidleitung 37 mit einer oder mehreren Druckanzeigevorrichtungen versehen sein. Dieses Hydrauliksystem wird als fertig zusammengesetzte und insbesondere bereits mit dem Hydraulikfluid 36 gefüllte Einheit hergestellt und als Ganzes sterilisiert geliefert.

Die Handhabung der erfindungsgemäßen Distraktionsvorrichtung ist hierdurch für den Operateur äußerst einfach. Gegebenenfalls im Anschluss an eine Sterilisation des Nicht-Hydrauliksystems, also der übrigen, nicht zu dem vorstehend erwähnten Hydrauliksystem gehörenden Bestandteile der Distraktionsvorrichtung, brauchen lediglich die beiden Kolben-/Zylinder-Anordnungen des sterilen Hydrauliksystems von unten in die Stützrohre 19 eingeschoben und mittels der Verriegelungseinrichtungen 77 gesichert sowie die Verstärkungseinsätze 25 mit den oberen Arbeitsabschnitten 11 von oben eingesetzt zu werden.

Abweichend von den beiden vorstehend beschriebenen Ausführungsformen kann die erfindungsgemäße Distraktionsvorrichtung auch lediglich eine einzige Kolben-/Zylinder-Anordnung 15, 17 mit einem einzigen Paar zungenförmigen Arbeitsabschnitten 11, 13 umfassen.

Bei den Bestandteilen der Kolben-/Zylinder-Anordnungen handelt es sich bevorzugt um im Spritzgussverfahren hergestellte Kunststoffteile, wohingegen zumindest die Stützrohre 19 und die Verstärkungseinsätze 21, 23, 25 aus einem Material höherer Biegesteifigkeit, beispielsweise aus Metall oder einem faserverstärkten Kunststoff, hergestellt sind, welche sich wegen ihrer Wiederverwendung im Spital zum Beispiel dampfsterilisieren lassen.

Die in den Fig. 1 bis 8 beschriebenen Distraktionsvorrichtungn dienen zum Aufspreizen des Knies, wie es im Einleitungsteil erläutert wurde, und ermöglichen das so genannte "Auspendeln" des Kniegelenks zur Bestimmung des durch die natürliche Spannung der Bänder bestimmten optimalen Abstandes zwischen Femur und Tibia unter gleichzeitiger Anzeige von für das betreffende Knie charakteristischen Messwerten für die momentanen Parameter Distraktionslänge und Distraktionskraft.

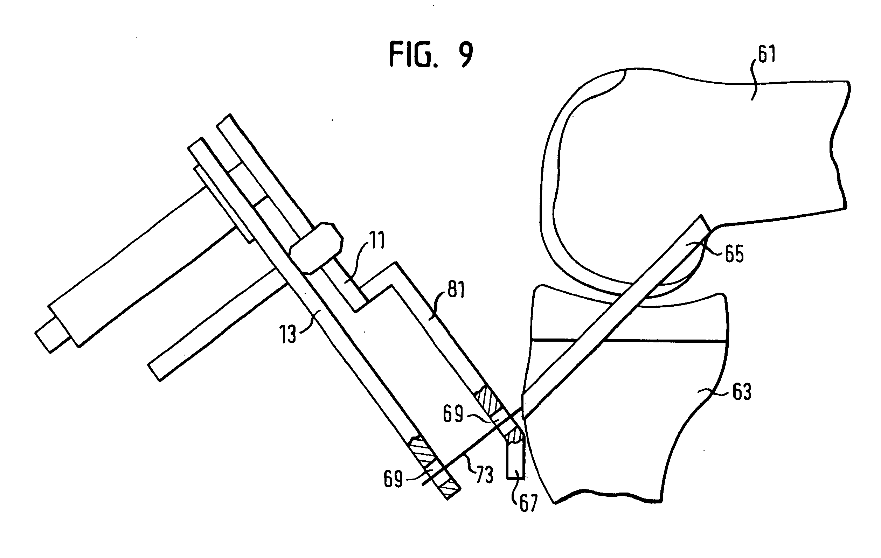

Die Fig. 9 bis 11 zeigen die Verwendung erfindungsgemäßer Distraktionsvorrichtungen beim Implantieren eines vorderen Kreuzbandes. Bei einer derartigen Operation wird das Band 65 in eine zuvor im Femur 61 ausgebildete Bohrung eingeschoben und dort mittels einer entlang der Mantellinie des Bandes 65 eingedrehten, nicht dargestellten Verdrängerschraube festgesetzt. Anschließend wird das Band durch eine zuvor in der Tibia 63 ausgebildete Bohrung nach vorne durchgezogen, um in dieser Tibiabohrung mittels einer weiteren, nicht dargestellten Verdrängerschraube festgesetzt zu werden.

Hier setzt jetzt die weitere Verwendung der erfindungsgemäßen Distraktionsvorrichtung ein:

Während es bislang üblich war, vor dem Festziehen der Tibia-Verdrängerschraube dem Band 65 eine vorgegebene Vorspannung unter Zuhilfenahme einer Federwaage zu verleihen, um das Band 65 unter dieser Vorspannung festzusetzen, wobei die Federwaage ohne weitere Abstützung nach außen gezogen wurde, wird nunmehr die Federwaage durch die erfindungsgemäße Distraktionsvorrichtung ersetzt, die sowohl eine einzige als auch - wie bei den vorstehend in Verbindung mit den Fig. 1 - 8 beschriebenen Ausführungsformen - zwei parallel angeordnete Kolben-/Zylinder-Anordnungen und damit obere Arbeitsabschnitte 11 aufweisen kann.

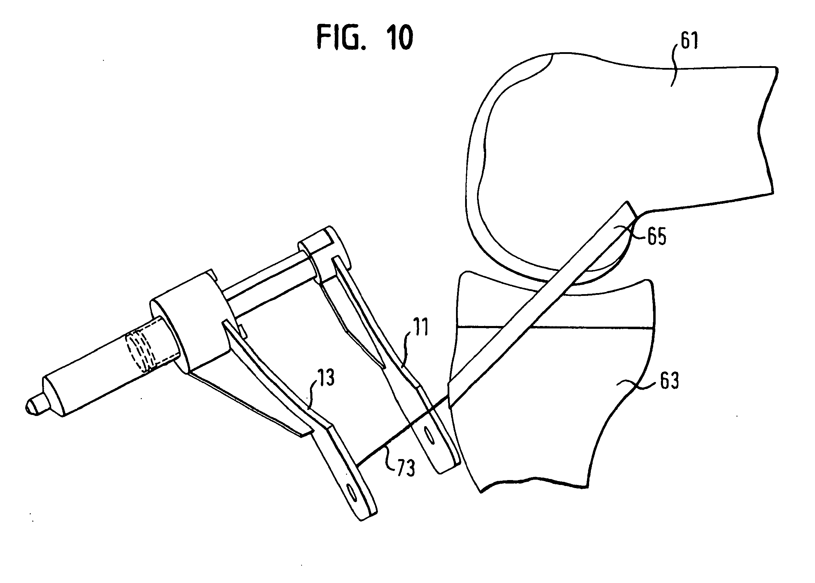

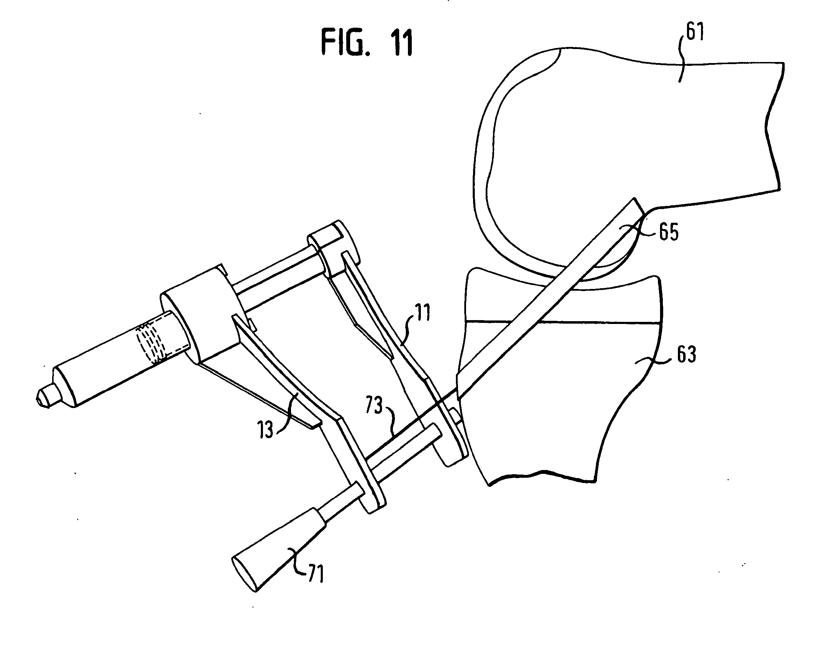

Im Folgenden wird der Einfachheit halber von einer "Mono-Variante" mit lediglich einer einzigen Kolben-/Zylinder-Anordnung ausgegangen, wie sie beispielsweise in den Fig. 10 und 11 dargestellt ist.

Während der Operation wird die Distraktionsvorrichtung mit ihrem oberen Arbeitsabschnitt 11, die - wie in Fig. 9 gezeigt - hierzu mit einem speziellen Aufsatz 81, dessen vorderes freies Ende von einem abgewinkelten oder abwinkelbaren Abstütz- und/oder Befestigungsabschnitt gebildet ist, versehen sein kann, an der Tibia 63 abgestützt. Das Band 65 und ggf. - wie in den Fig. 9-11 gezeigt - ein mit dem Band 65 verbundenes Zugelement 73 beispielsweise in Form eines Drahtes wird durch in beiden Arbeitsabschnitten 11, 13 ausgebildete Aussparungen 69 hindurch gezogen und an dem unteren Arbeitsabschnitt 13 befestigt. Die Aussparungen 69 sind dabei derart bemessen, dass ein in Fig. 11 schematisch dargestellter Schraubendreher 71 mit aufgesetzter Verdrängerschraube entlang dem Band 65 eingebracht werden kann, um das die richtige Vorspannung aufweisende Band 65 in der Tibia 63 festzusetzen.

Mittels der erfindungsgemäßen Distraktionsvorrichtung ist es in vorteilhafter Weise möglich, vor dem endgültigen Festsetzen des Bandes 65 in der Tibia 63 entweder bei allen möglichen oder zumindest bei einigen ausgewählten Stellungen, d.h. Beugungswinkeln, des Knies die Bandspannung zu messen bzw. ein Maß für die Bandspannung zu ermitteln, um ggf. durch Variieren der Bandlänge eine Bandspannung einzustellen, die für alle möglichen Beugungswinkel und damit für den normalen Bewegungsablauf des Kniegelenks geeignet ist. Das Maß für die Bandspannung wird mit einer Druckanzeigeeinrichtung ermittelt, wie sie beispielsweise in Verbindung mit den Ausführungsformen der Fig. 1 - 8 beschrieben wurde, während das Maß für die Bandlänge durch die Distraktionslänge gegeben ist, die an einer Anzeigeeinrichtung, z.B. in Form einer Skala, abgelesen werden kann, wie sie ebenfalls beispielsweise in Verbindung mit den Ausführungsformen der Fig. 1 - 8 beschrieben wurde.

Während der Ermittlung der optimalen Länge des zu implantierenden Bandes 65 kann der obere Arbeitsabschnitt 11 beispielsweise mit Hilfe von Knochennägeln an der Tibia 63 befestigt werden, um für definierte geometrische Verhältnisse zu sorgen.

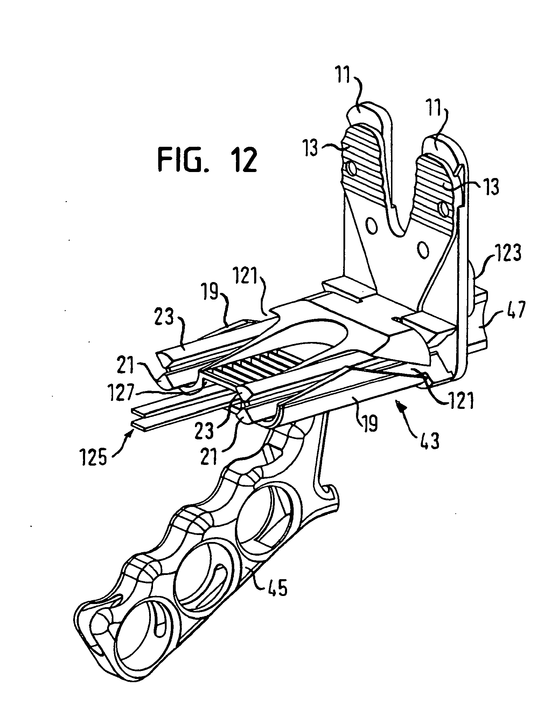

Das nachstehend anhand der Fig. 12 bis 24 beschriebene weitere Ausführungsbeispiele einer erfindungsgemäßen Distraktionsvorrichtung entspricht hinsichtlich des Prinzips der Verstärkung der hier nicht dargestellten Kolben-/Zylinder-Anordnungen sowie hinsichtlich weiterer Aspekte, die sich aus der nachstehenden Beschreibung ergeben, grundsätzlich dem anhand der Fig. 1 bis 4 beschriebenen ersten Ausführungsbeispiel.

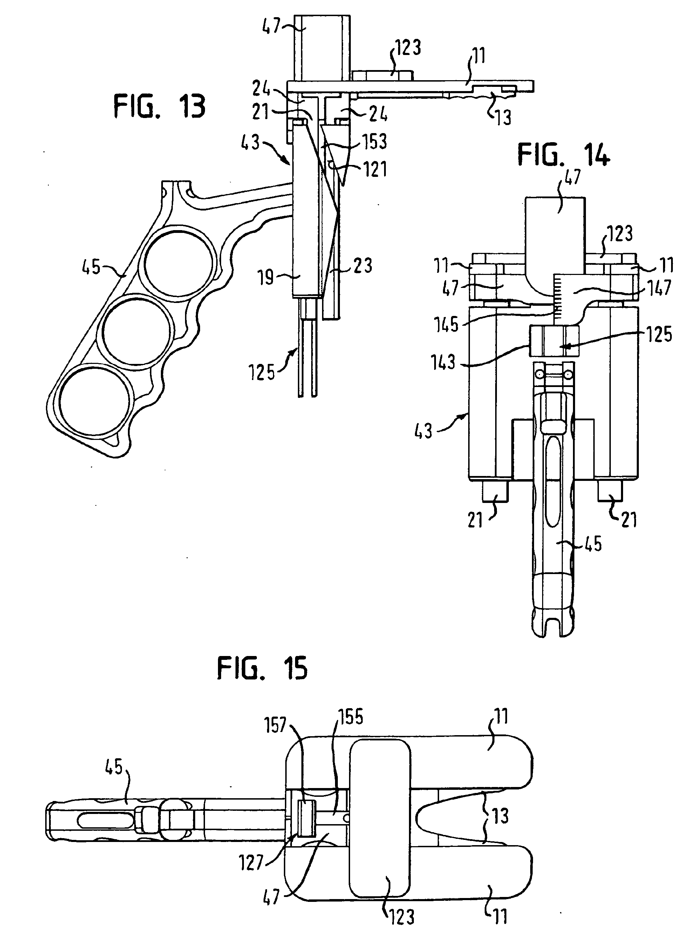

Die beiden oberen Arbeitsabschnitte 11 oder Zungen (vgl. insbesondere Fig. 12 - 15 sowie 23 und 24) sind im Bereich ihres hinteren Endes fest mit einem hinteren Verstärkungseinsatz 21 verbunden. Separate Verstärkungseinsätze 23 dienen zur Komplettierung der Kolbenverstärkung entsprechend dem ersten Ausführungsbeispiel (Fig. 1). Wie bereits erwähnt, sind der Kolben und der Zylinder der Kolben-/Zylinder-Anordnungen, die jeweils wiederum von einer handelsüblichen Einwegspritze gebildet werden, nicht dargestellt. Insbesondere in Fig. 13 ist jedoch der Freiraum 153 für den Kolben einschließlich des Bereiches für den Kopfabschnitt oder Pilz des Kolbens zwischen den Flanschabschnitten 24 der Verstärkungseinsätze 21, 23 zu erkennen.

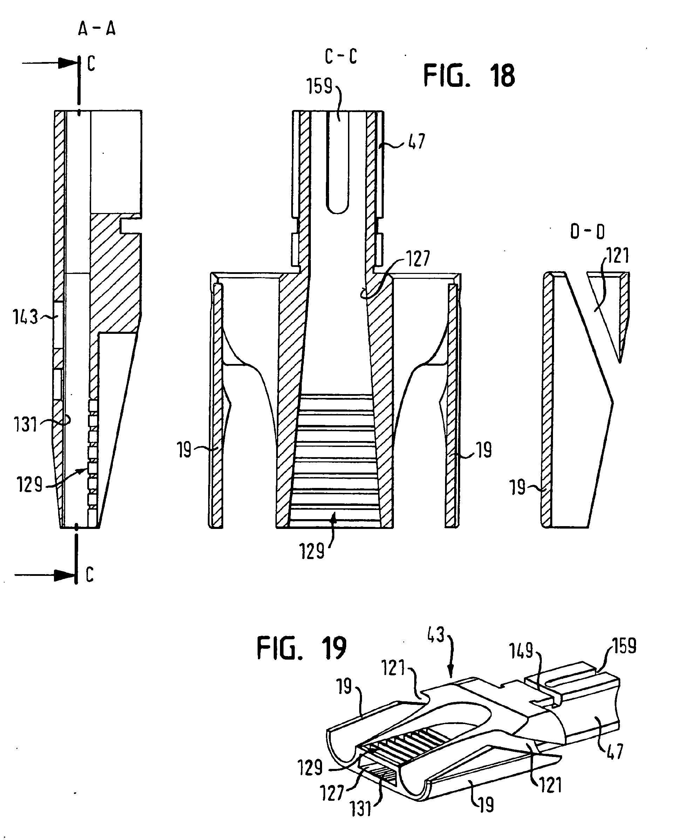

Im zusammengesetzten Zustand der Distraktionsvorrichtung ist der Kolben einschließlich der Verstärkungseinsätze 21, 23 in den Zylinder der jeweiligen Kolben-/Zylinder-Anordnung eingesteckt, der wiederum in eine betreffende Stützaufnahme 19 eingeführt ist, die an einem zentralen Tragkörper 43 ausgebildet ist, der nachstehend näher beschrieben wird. Die Stützaufnahmen 19 sind jeweils teilrohrförmig ausgebildet und in Form eines von unten schräg aufgeschnittenen Stützrohres vorgesehen (Fig. 19). Der weggeschnittene Bereich weist dabei nach vorne, d.h. in Richtung der freien Enden der Arbeitsabschnitte 11, 13, so dass in die entgegengesetzte Richtung, d.h. nach hinten, eine Abstützung über die gesamte Länge der Stützaufnahmen 19 gegeben ist.

Die Stützaufnahmen 19 sind jeweils mit einer schlitzförmigen Einlegeöffnung 121 versehen, die sich von der oberen Einstecköffnung bis in den oberen Bereich der durch das Aufschneiden schräg verlaufenden unteren Öffnung der Stützaufnahme 19 erstreckt.

Die seitlichen Einlegeöffnungen 121 gestatten es in vorteilhafter Weise, beim Zusammensetzen der Distraktionsvorrichtung den Zylinder mit unten angeschlossener Fluidleitung von oben in die jeweilige Stützaufnahme 19 einzuführen, indem die Fluidleitung einfach seitlich über die Einlegeöffnung 121 eingelegt wird. Die Fluidleitung kann somit stets fest mit dem Zylinder verbunden bleiben, wobei es jedoch nicht - wie etwa bei dem Ausführungsbeispiel der Fig. 7 - erforderlich ist, den Zylinder von unten einzuführen und eine Verriegelungseinrichtung vorzusehen.

Der einen etwa Y-förmigen Umriss aufweisende untere Arbeitsabschnitt 13, dessen Schenkel von den beiden unteren Zungen gebildet sind, ist über sein hinteres Ende in einem am Tragkörper 43 ausgebildeten Schlitz 149 (Fig. 19) befestigt.

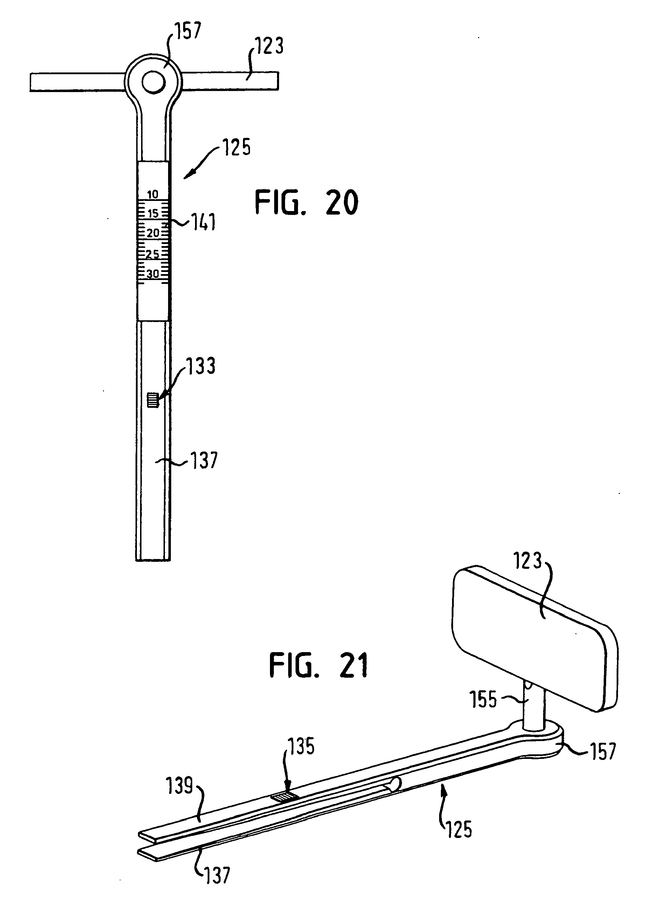

Der Tragkörper 43 weist ferner einen sich parallel zur Distraktionsachse erstreckenden, durchgehenden Verstellkanal 127 auf, der zwischen den beiden Stützaufnahmen 19 hindurch verläuft. Der Verstellkanal 127 dient zur Aufnahme einer Tragstange 125 eines außerdem eine Plattform 123 aufweisenden Trägers, der nachstehend in Verbindung mit den Fig. 20 bis 22 näher beschrieben wird.

In der Rückwand des Tragkörpers 43 ist ein Ablesefenster 143 zum Verstellkanal 127 ausgebildet, über welches die Rückseite der in den Verstellkanal 127 eingeführten Tragstange 125 sichtbar und eine an dieser Rückseite angebrachte Skala 141 (Fig. 20) ablesbar ist. Ein Handgriff 45 der Distraktionsvorrichtung ist unterhalb des Ablesefensters 143 mit der Rückwand des Tragkörpers 43 verbunden.

Der Verstellkanal 127 und die Tragstange 125 sind mit Profilierungen 129, 131, 133, 135 in Form von Rippenstrukturen versehen, auf die nachstehend näher eingegangen wird.

Entsprechend dem Ausführungsbeispiel der Fig. 1 bis 4 ist ein sich längs der Distraktionsachse erstreckender Funktionsblock 47 vorgesehen. Der Funktionsblock 147 ist ein integraler Bestandteil des zentralen Tragkörpers 43.

Der Funktionsblock 47 dient u.a. zur Führung der oberen Arbeitsabschnitte 11. Ferner sind in den Funktionsblock 47 ein Schwenkkopf 157 der Tragstange 125 sowie ein sich senkrecht zur Tragstange 125 erstreckendes Verbindungsstück 155 aufgenommen (Fig. 15), über welches die Plattform 123 mit der Tragstange 125 verbunden ist.

Die Plattform 123 ist unmittelbar vor dem Funktionsblock 47 angeordnet. Die Breite der Plattform 123 ist geringfügig kleiner als die Gesamtbreite des oberen Arbeitsabschnitts 11, während die Tiefe der Plattform 123 derart bemessen ist, dass - vom Funktionsblock 47 aus gesehen - die Plattform endet, bevor sich der untere Arbeitsabschnitt in die beiden Arbeitsabschnitte 13 teilt.

Hierdurch befindet sich die Plattform 123 bei zum Aufspreizen des Knies zwischen Femur und Tibia eingebrachten Arbeitsabschnitten 11, 13 außerhalb der Projektion der Tibia längs der Distraktionsachse.

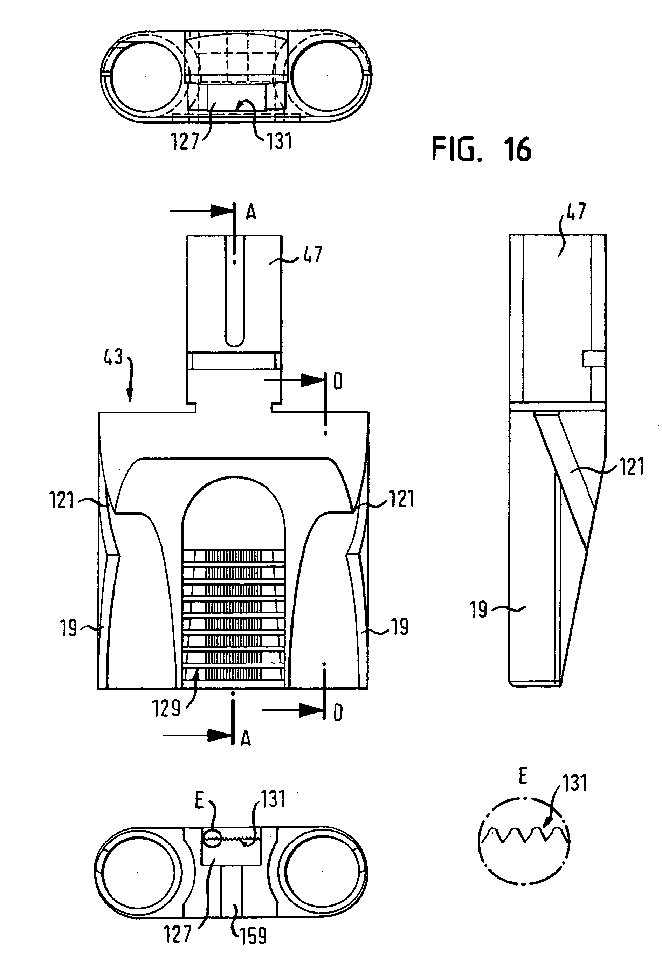

Den Fig. 16 bis 19 sind insbesondere die Anordnung und die Ausgestaltung der mit der Tragstange 125 zusammenwirkenden Profilierungen 129, 131 zu entnehmen, die an den Innenseiten des Verstellkanals 127 ausgebildet sind.

Eine Profilierung 129 in Form von sich quer zur Distraktionsachse erstreckenden Rippen oder Stegen, die im unteren Bereich des Verstellkanals 127 ausgebildet sind, dient zur Fixierung der Tragstange 125 in unterschiedlichen Höhenlagen. Wie insbesondere Fig. 18 zeigt, ist der Verstellkanal 127 ausgehend von dem Höhenniveau der oberen Einführöffnungen der Stützaufnahmen 19 nach unten derart erweitert, dass die Breite des Verstellkanals 127 stetig zunimmt. In dem unteren Bereich dieser Erweiterung des Verstellkanals 127, der im Querschnitt die Form eines schlanken Trapezes aufweist, ist die Querprofilierung 129 ausgebildet.

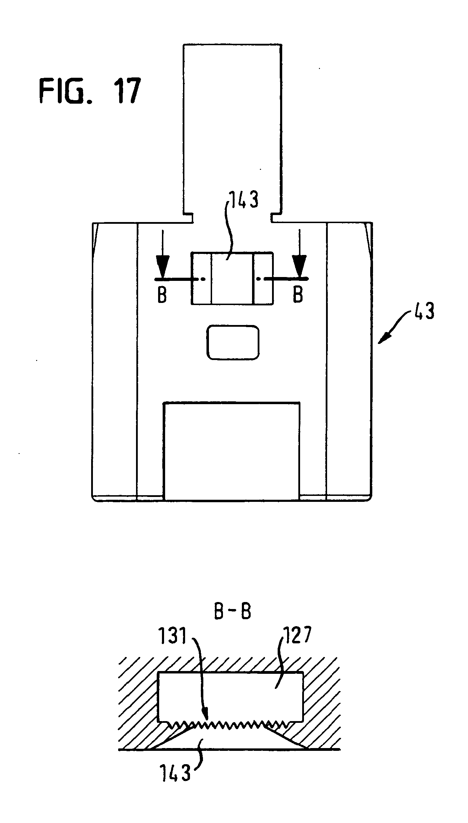

An der gegenüberliegenden Innenwand (Fig. 18, linke Darstellung) ist der Verstellkanal 127 mit einer Längsprofilierung 131 in Form von sich parallel zur Distraktionsachse erstreckenden Rippen versehen (Fig. 17). Diese Profilierung 131 dient dazu, die Tragstange 125 in unterschiedlichen Schrägstellungen zu fixieren, worauf nachstehend näher eingegangen wird.

Die Fig. 20 bis 22 zeigen, dass die Tragstange 125 bereichsweise als Gabel mit zwei Gabelarmen 137, 139 ausgebildet ist. Der Abstand der Gabelarme 137, 139 voneinander ist derart auf den Abstand der mit den Profilierungen 129, 131 versehenen Innenseiten des Verstellkanals 127 des Tragkörpers 43 abgestimmt, das bei in den Verstellkanal 127 eingeführter Tragstange 125 an den voneinander abgewandten Außenseiten der Gabelarme 137, 139 ausgebildete Profilierungen 133, 135 sich in Eingriff mit den Profilierungen 131, 129 des Verstellkanals 127 befinden.

Durch Zusammendrücken der beiden Gabelarme 137, 139 gegen deren Rückstellkraft kann der Eingriff zwischen den Profilierungen gelöst und die Tragstange 125 im Verstellkanal 127 relativ zum Tragkörper 43 bewegt werden.

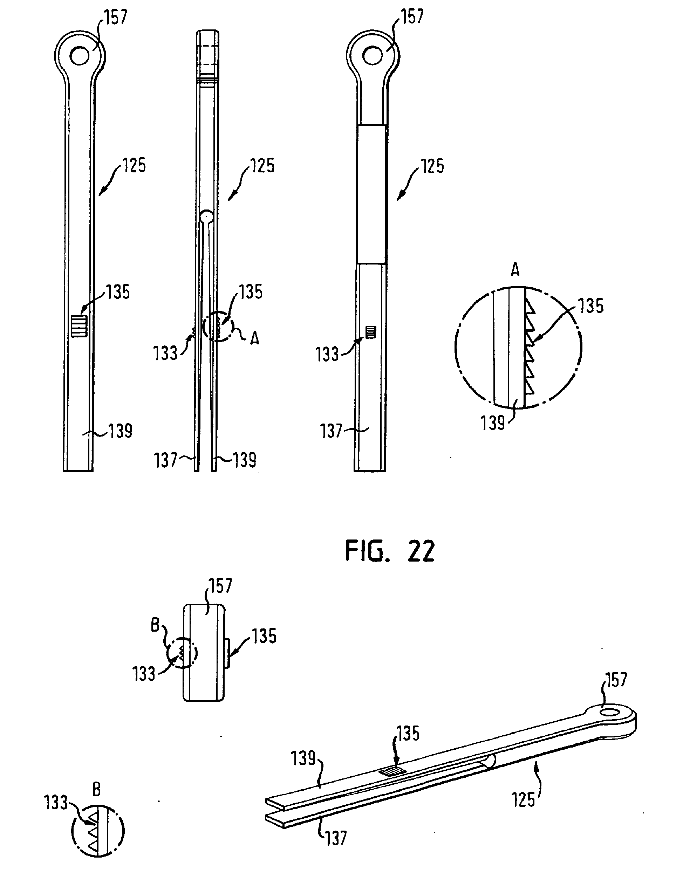

Insbesondere Fig. 22 ist zu entnehmen, dass die Profilierung 133 der Tragstange 125 in Form von Längsrippen vorgesehen ist, wohingegen die auf der gegenüberliegenden Seite ausgebildete Profilierung 135 Querrippen umfasst, die - wie das Detail A oben rechts in Fig. 22 zeigt - eine Sägezahnform derart aufweisen, dass Bewegungen der Tragstange 125 aus dem Verstellkanal 127 heraus ohne Zusammendrücken der Gabelarme 137, 139 möglich sind, Bewegungen der Tragstange 125 nach unten in den Verstellkanal 127 hinein dagegen blockiert und nur bei zusammengedrückten Gabelarmen 137, 139 möglich sind. Insofern wirken die Tragstange 125 und der Verstellkanal 127 aufgrund ihrer Profilierungen 135, 129 bezüglich Bewegungen parallel zur Distraktionsachse nach Art einer Ratsche zusammen.

Der Durchmesser des eine teilkreisförmige Außenform aufweisenden oberen Schwenkkopfes 157 der Tragstange 125, mit welchem die Plattform 123 über das Verbindungsstück 155 verbunden ist, entspricht der konstanten Breite des Verstellkanals 127 oberhalb der Erweiterung.

Folglich kann die Tragstange 125 um den im Verstellkanal 127 geführten Schwenkkopf 157 in der von den Mittelachsen der beiden teilrohrförmigen Stützaufnahmen 19 festgelegten Ebene verschwenkt werden, wobei die Schwenkachse durch das die Plattform 123 mit dem Schwenkkopf 157 verbindende Verbindungsstück 155 hindurch verläuft, das sich in einer Aussparung 159 des Funktionsblocks 47 drehen kann (Fig. 16, 18, 19). Bewegungen des in den Funktionsblock 47 aufgenommenen Schwenkkopfes 157 quer zur Distraktionsachse sind nicht möglich.

Bei zusammengedrückten Gabelarmen 137, 139 und damit außer Eingriff gelangten Profilierungen 129, 135 bzw. 131, 133 kann somit die Tragstange 125 im Verstellkanal 127 gewissermaßen um ihren Schwenkkopf 157 "pendeln", wobei durch die Erweiterung des Verstellkanals 127 nach unten für den hierfür erforderliche Bewegungsspielraum für die Tragstange 125 gesorgt ist.

Das Verschwenken der Tragstange 125 im Verstellkanal 127 hat ein Verkippen der Plattform 123 aus einer Neutralposition senkrecht zur Distraktionsachse heraus zur Folge. Die Längsprofilierungen 131, 133 ermöglichen ein Fixieren der Tragstange 125 im aus einer parallelen Ausrichtung zur Distraktionsachse ausgelenkten Zustand, d.h. eine Schräglage der Plattform 123 kann mittels der Profilierungen 131, 133 fixiert werden. Die an der Tragstange 125 ausgebildeten Profilierungen 133, 135, insbesondere die Quer-Profilierung 135, sind dabei derart tief und in ihrer Breite bzw. Länge bemessen, dass die Tragstange 125 trotz Schrägstellung einrastet.

Der Tragkörper 43 ist in seinem unteren Bereich mit einer nicht dargestellten Anzeigeeinrichtung in Form einer Skala versehen, an welcher ein Maß für eine jeweilige Schwenkstellung der Tragstange 125 und damit für die betreffende Schrägstellung der Plattform 123 abgelesen werden kann.

In Fig. 20 ist die bereits erwähnte, an der Außenseite des hinteren Gabelarmes 137 ausgebildete Skala 141 dargestellt, die über das in der Rückwand des Tragkörpers 43 ausgebildete Ablesefenster 143 von außen für den Operateur sichtbar ist (Fig. 14). Über diese Skala 141 kann die Höhenlage der Tragstange 125 und damit der Plattform 123 bezüglich des Tragkörpers 43 bestimmt werden.

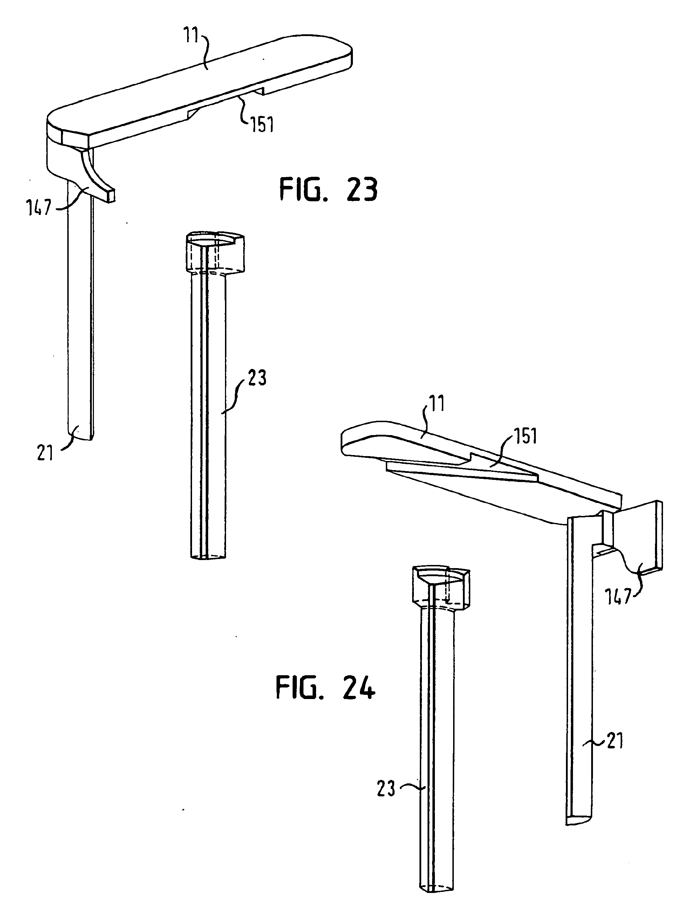

Die Fig. 23 und 24 zeigen die oberen Arbeitsabschnitte 11 sowie die Verstärkungseinsätze 21, 23. Dargestellt sind ferner nach innen vorstehende Laschen 147, deren Rückseiten senkrecht zu der von den Arbeitsabschnitten 11 gebildeten Plattform verlaufen. Wie Fig. 14 zeigt, ist die für den Operateur während der Operation sichtbare Rückseite der mit dem rechten Arbeitsabschnitt verbundenen Lasche 147 (Fig. 24) mit der bereits erwähnten Skala 145 versehen, über welche die Höhenlage des oberen Arbeitsabschnitts 11 in Bezug auf den Tragkörper 43 abgelesen werden kann.

Ferner sind Fig. 23 und 24 an den Unterseiten der oberen Arbeitsabschnitte 11 ausgebildete Aussparungen 151 dargestellt, in die an der Oberseite des unteren Arbeitsabschnitts 13 ausgebildete Erhöhungen eingreifen, wenn die beiden Arbeitsabschnitten 11, 13 vollständig zusammengefahren sind (Fig. 12 und 13).

Des Weiteren ist es möglich, auf der Plattform 123 weitere Vorrichtungen wie beispielsweise eine um einen im Femur eingesetzten Marknagel schwenkbare Schwenkvorrichtung anzubringen, die ein Ausbalancieren der Bänderspannung unter einer vorgegebenen, an der Hydraulik der von den Einwegspritzen gebildeten Kolben-/Zylinder-Anordnungen ablesbaren Vorspannung erlaubt.

### Bezugszeichenliste

- 11: oberer Arbeitsabschnitt
- 13: unterer Arbeitsabschnitt
- 15: Kolben
- 16: Kolbenabschnitt
- 17: Zylinder
- 19: Stützaufnahme, Stützrohr
- 21: Verstärkungseinsatz
- 23: Verstärkungseinsatz
- 24: Flanschabschnitt
- 25: Verstärkungseinsatz
- 27: Kolbenstange
- 29: Kammer
- 31: Rippenstruktur
- 33: Fluidzuführeinrichtung
- 35: Spritze
- 36: Hydraulikfluid
- **37**: **Fluidleitung**
- 39: Druckanzeigeeinrichtung
- 41: Schaltventil
- 43: Tragkörper
- 44: Ringschulter
- 45: Handgriff
- 47: Funktionsblock
- 49: Führungsabschnitt
- 51: Anschlagfläche
- 53: Anzeigeeinrichtung, Skala
- 55: Anschlussabschnitt
- 57: Kopfabschnitt, Pilz
- 59: Lasche
- 61: Femur
- 63: Tibia
- 65: Kreuzband
- 67: Abstütz- und/oder Befestigungsabschnitt
- 69: Aussparung
- 71: Fixierwerkzeug, Schraubendreher
- 73: Zugelement
- 77: Verriegelungseinrichtung
- 79: gabelförmiger Verriegelungsabschnitt
- 81: Aufsatz
- 121: Einlegeöffnung
- 123: Plattform
- 125: Tragstange
- 127: Verstellkanal
- 129: Profilierung
- 131: Profilierung
- 133: Profilierung
- 135: Profilierung
- 137: Gabelarm
- 139: Gabelarm
- 141: Anzeigeeinrichtung, Skala
- 143: Ablesefenster
- 145: Anzeigeeinrichtung, Skala
- 147: Lasche
- 149: Schlitz
- 151: Aussparung
- 153: Freiraum für Kolben
- 155: Verbindungsstück
- 157: Schwenkkopf
- 159: Aussparung für Verbindungsstück

## Patentansprüche

1. Hydraulische Distraktionsvorrichtung für Operationen, insbesondere für Knieoperationen, mit wenigstens einem Paar relativ zueinander verstellbarer Arbeitsabschnitte (11, 13) und zumindest einer Kolben-/Zylinder-Anordnung (15, 17), deren Kolben (15) mit dem einen Arbeitsabschnitt (11) und deren Zylinder (17) mit dem anderen Arbeitsabschnitt (13) gekoppelt ist,
**dadurch gekennzeichnet,**
**dass** die Kolben-/Zylinder-Anordnung (15, 17) auswechselbar und aus Kunststoff hergestellt ist, wobei die Koppelung des Kolbens (15) und des Zylinders (17) mit den Arbeitsabschnitten (11, 13) jeweils lösbar ist, und
**dass** die Kolben-/Zylinder-Anordnung (15, 17) durch eine äußere, den Zylinder (17) von außen abstützende Stützaufnahme (19) sowie durch wenigstens einen inneren, zusammen mit dem Kolben (15) in den Zylinder (17) einführbaren Verstärkungseinsatz (21, 23, 25) versteift ist.

2. Distraktionsvorrichtung nach Anspruch 1,
**dadurch gekennzeichnet,**
**dass** der Kolben (15) und der Zylinder jeweils im Spritzgussverfahren hergestellte Einwegartikel sind.

3. Distraktionsvorrichtung nach Anspruch 1 oder 2,
**dadurch gekennzeichnet,**
**dass** der Kolben (15) und der Verstärkungseinsatz (21, 23, 25) im zusammengesetzten Zustand eine kompakte Kolbeneinheit bilden, die zumindest über einen Teilumfangsbereich das Innere des Zylinders (17) vollständig ausfüllt.

4. Distraktionsvorrichtung nach einem der vorhergehenden Ansprüche,
**dadurch gekennzeichnet,**
**dass** die Kolben-/Zylinder-Anordnung (15, 17) von einer handelsüblichen Einwegspritze gebildet ist.

5. Distraktionsvorrichtung nach einem der vorhergehenden Ansprüche,
**dadurch gekennzeichnet,**
**dass** ein zwischen der Innenwand des Zylinders (17) und einer Kolbenstange (27) vorhandener Freiraum durch den Verstärkungseinsatz (21, 23) zumindest teilweise ausgefüllt ist.

6. Distraktionsvorrichtung nach einem der vorhergehenden Ansprüche,
**dadurch gekennzeichnet,**
**dass** wenigstens eine im Zylinder (17) vorhandene Kammer (29), die durch eine bis an die Innenwand des Zylinders (17) reichende, insbesondere im Querschnitt kreuz- oder X-förmige Rippenstruktur (31) einer Kolbenstange (27) begrenzt ist, durch den Verstärkungseinsatz (21, 23) zumindest teilweise ausgefüllt ist.

7. Distraktionsvorrichtung nach Anspruch 6,
**dadurch gekennzeichnet,**
**dass** wenigstens ein Paar einander gegenüberliegender Kammern jeweils durch einen Verstärkungseinsatz (21, 23) zumindest teilweise ausgefüllt ist.

8. Distraktionsvorrichtung nach einem der Ansprüche 1 bis 5,
**dadurch gekennzeichnet,**
**dass** der Verstärkungseinsatz (25) das Innere einer rohrförmigen, an der Innenwand des Zylinders (17) geführten Kolbenstange (27) zumindest über einen Teilumfangsbereich vollständig ausfüllt.

9. Distraktionsvorrichtung nach einem der vorhergehenden Ansprüche,
**dadurch gekennzeichnet,**
**dass** die Kolben-/Zylinder-Anordnung (15, 17) im zusammengesetzten Zustand mit den Arbeitsabschnitten (11, 13) koppelbar ist.

10. Distraktionsvorrichtung nach einem der vorhergehenden Ansprüche,
**dadurch gekennzeichnet,**
**dass** wenigstens ein Verstärkungseinsatz (21, 25) fest mit dem einen Arbeitsabschnitt (11) verbunden ist.

11. Distraktionsvorrichtung nach einem der vorhergehenden Ansprüche,
**dadurch gekennzeichnet,**
**dass** wenigstens ein Verstärkungseinsatz (23) in Form eines separaten Bauteils vorgesehen ist.

12. Distraktionsvorrichtung nach einem der vorhergehenden Ansprüche,
**dadurch gekennzeichnet,**
**dass** die Stützaufnahme (19) für den Zylinder (17) und der mit dem Zylinder (17) koppelbare Arbeitsabschnitt (13) fest mit einem Tragkörper (43) verbunden sind.

13. Distraktionsvorrichtung nach einem der vorhergehenden Ansprüche,
**dadurch gekennzeichnet,**
**dass** auf der den Arbeitsabschnitten (11, 13) gegenüberliegenden Seite der Kolben-/Zylinder-Anordnung (15, 17) ein Handgriff (45) vorgesehen ist, der bevorzugt fest mit einem Tragkörper (43) verbunden ist.

14. Distraktionsvorrichtung nach einem der vorhergehenden Ansprüche,
**dadurch gekennzeichnet,**
**dass** der mit dem Zylinder (17) koppelbare Arbeitsabschnitt (13) fest mit einem sich parallel zur Distraktionsrichtung erstreckenden Funktionsblock (47) verbunden ist, der als Verdreh- und/oder Auslenksicherung für den mit dem Kolben (15) koppelbaren Arbeitsabschnitt (11) ausgebildet ist.

15. Distraktionsvorrichtung nach Anspruch 14,
**dadurch gekennzeichnet,**
**dass** der Funktionsblock (47) einen von einer Kreisform abweichenden Außenquerschnitt aufweist und zumindest über einen Teilumfangsbereich von einem Führungsabschnitt (49) des Arbeitsabschnitts (11) verdrehsichernd umgriffen ist.

16. Distraktionsvorrichtung nach Anspruch 14 oder 15,
**dadurch gekennzeichnet,**
**dass** der Funktionsblock (47) eine geringfügig gegenüber der Distraktionsrichtung geneigte, in Richtung zunehmender Distraktionslänge auf die Längsachse der Kolben-/Zylinder-Anordnung (15, 17) zu laufende Anschlagfläche (51) aufweist, die mit dem Arbeitsabschnitt (11) auslenkungssichernd zusammenwirkt.

17. Distraktionsvorrichtung nach einem der Ansprüche 14 bis 16,
**dadurch gekennzeichnet,**
**dass** der Funktionsblock (47) mit einer Anzeigeeinrichtung (53), insbesondere in Form einer an einer Außenseite angebrachten Skala, versehen ist, an der ein Maß für die Distraktionslänge ablesbar ist.

18. Distraktionsvorrichtung nach einem der vorhergehenden Ansprüche,
**dadurch gekennzeichnet,**
**dass** die Stützaufnahme (19) und/oder der Verstärkungseinsatz (21, 23, 25) aus einem Material mit einer höheren Biegesteifigkeit als der Kunststoff der Kolben-/Zylinder-Anordnung (15, 17) hergestellt sind, insbesondere aus Metall oder einem faserverstärkten Kunststoff.

19. Distraktionsvorrichtung nach einem der vorhergehenden Ansprüche,
**dadurch gekennzeichnet,**
**dass** die Kolben-/Zylinder-Anordnung (15, 17) mit einer Anzeigeeinrichtung, insbesondere in Form einer am Zylinder angebrachten Skala, versehen ist.

20. Distraktionsvorrichtung nach einem der vorhergehenden Ansprüche,
**dadurch gekennzeichnet,**
**dass** eine Fluidzuführeinrichtung (33) für die Kolben-/Zylinder-Anordnung (15, 17) eine von Hand betätigbare Spritze (35) umfasst, insbesondere eine Einwegspritze.

21. Distraktionsvorrichtung nach einem der vorhergehenden Ansprüche,
**dadurch gekennzeichnet,**
**dass** an eine die Kolben-/Zylinder-Anordnung (15, 17) mit einer Fluidzuführeinrichtung (33) verbindende Fluidleitung (37) wenigstens eine Druckanzeigeeinrichtung (39) angeschlossen ist.

22. Distraktionsvorrichtung nach einem der vorhergehenden Ansprüche,
**dadurch gekennzeichnet,**
**dass** die Kolben-/Zylinder-Anordnung (15, 17) ein Bestandteil eines geschlossenen, als eine Einheit handhabbaren, insbesondere sterilisierbaren, Hydrauliksystems ist, das zusätzlich zumindest eine Fluidzuführeinrichtung (33), eine die Kolben-/Zylinder-Anordnung (15, 17) mit der Fluidzuführeinrichtung (33) verbindende Fluidleitung (37) sowie ein Hydraulikfluid, insbesondere Wasser, umfasst und als Ganzes mit den Arbeitsabschnitten (11, 13) koppelbar ist.

23. Distraktionsvorrichtung nach einem der vorhergehenden Ansprüche,
**dadurch gekennzeichnet,**
**dass** wenigstens zwei im mit den Arbeitsabschnitten (11, 13) gekoppelten Zustand in parallelen Richtungen wirksame Kolben-/Zylinder-Anordnungen (15, 17) vorgesehen sind.

24. Distraktionsvorrichtung nach Anspruch 23,
**dadurch gekennzeichnet,**
**dass** die Kolben-/Zylinder-Anordnungen (15, 17) während der Benutzung an eine gemeinsame Fluidzuführeinrichtung (33) angeschlossen sind.

25. Distraktionsvorrichtung nach Anspruch 23 oder 24,
**dadurch gekennzeichnet,**
**dass** durch in einer die Kolben-/Zylinder-Anordnungen (15, 17) mit einer Fluidzuführeinrichtung (33) verbindenden Fluidleitung (37) angeordnete Schaltventile (41) die Kolben-/ Zylinder-Anordnungen (15, 17) unabhängig voneinander jeweils wahlweise mit der Fluidzufuhr verbindbar oder von der Fluidzufuhr trennbar sind.

26. Distraktionsvorrichtung nach einem der vorhergehenden Ansprüche,
**dadurch gekennzeichnet,**
**dass** zum Einstellen von Sollabständen zwischen Femur (61) und Tibia (63) die Arbeitsabschnitte (11, 13) einen zwischen Femur (61) und Tibia (63) einbringbaren und sich im Wesentlichen senkrecht zur Distraktionsrichtung erstreckenden Aufspreizabschnitt bilden.

27. Distraktionsvorrichtung nach einem der vorhergehenden Ansprüche,
**dadurch gekennzeichnet,**
**dass** zur Bestimmung der Bandspannung und/oder Bandlänge bei der Implantation von vorderen Kreuzbändern (65) ein oberer Arbeitsabschnitt (11) mit einem Abstütz- und/oder Befestigungsabschnitt (67, 81) versehen ist, über den die Vorrichtung an der Tibia (63) abstützbar und/oder befestigbar ist.

28. Distraktionsvorrichtung nach Anspruch 27,
**dadurch gekennzeichnet,**
**dass** die Arbeitsabschnitte (11, 13) jeweils mit wenigstens einer Aussparung (69) zum Hindurchführen eines Fixierwerkzeugs (71) und eines Fixierelementes zum Fixieren des Bandes (65) in der Tibia (63) versehen sind.

29. Distraktionsvorrichtung nach Anspruch 27 oder 28,
**dadurch gekennzeichnet,**
**dass** der obere Arbeitsabschnitt (11) mit wenigstens einer Aussparung (69) zum Hindurchführen des Bandes (65) und/oder eines mit dem Band (65) verbundenen Zugelementes (73) versehen ist.

30. Distraktionsvorrichtung nach einem der Ansprüche 27 bis 29,
**dadurch gekennzeichnet,**
**dass** ein unterer Arbeitsabschnitt (13) mit wenigstens einem Befestigungsabschnitt zum Befestigen des Bandes (65) oder eines mit dem Band (65) verbundenen Zugelementes (73) versehen ist.

31. Distraktionsvorrichtung nach einem der vorhergehenden Ansprüche,
**dadurch gekennzeichnet,**
**dass** die Arbeitsabschnitte (11, 13) jeweils als zungenförmige Plattformen ausgebildet sind, die im zusammengesetzten Zustand zumindest im Wesentlichen parallel zueinander verlaufen.

32. Distraktionsvorrichtung nach einem der vorhergehenden Ansprüche,
**dadurch gekennzeichnet,**
**dass** die Stützaufnahme (19) für den Zylinder zumindest teilrohrförmig ausgebildet und der Zylinder längs der Rohrachse in die Stützaufnahme (19) einführbar ist, wobei die Stützaufnahme (19) zum seitlichen Einlegen einer mit dem Zylinder verbundenen Fluidleitung eine insbesondere schlitzförmige Einlegeöffnung (121) aufweist.

33. Distraktionsvorrichtung nach einem der vorhergehenden Ansprüche,
**dadurch gekennzeichnet,**
**dass** ein Träger (123, 125) für eine Schnittlehre, insbesondere einen Schnittblock, vorgesehen ist, der mit einem Tragkörper (43) koppelbar ist, mit dem die Stützaufnahme (19) für den Zylinder und der mit dem Zylinder koppelbare Arbeitsabschnitt (13) verbunden sind.

34. Distraktionsvorrichtung nach Anspruch 33,
**dadurch gekennzeichnet,**
**dass** der Träger eine als Auflage für die Schnittlehre dienende Plattform (123) und eine mit dem Tragkörper (43) koppelbare Tragstange (125) umfasst, die an ihrem einen Ende die Plattform (123) trägt und in einen Verstellkanal (127) des Tragkörpers (43) einführbar ist, in welchem die Tragstange (125) relativ zum Tragkörper (43) verstellbar und in einer Vielzahl von insbesondere diskret verteilten Positionen am Tragkörper (43) fixierbar ist.

35. Distraktionsvorrichtung nach Anspruch 34,
**dadurch gekennzeichnet,**
**dass** die Tragstange (125) und die Innenwand des Verstellkanals (127) mit zur Fixierung ihrer Relativlage zusammenwirkenden, insbesondere rippenförmigen Profilierungen (129, 131, 133, 135) versehen sind.

36. Distraktionsvorrichtung nach Anspruch 34 oder 35,
**dadurch gekennzeichnet,**
**dass** die Verstellanordnung aus Tragstange (125) und Verstellkanal (127) selbsthemmend ausgebildet ist.

37. Distraktionsvorrichtung nach einem der Ansprüche 34 bis 36,
**dadurch gekennzeichnet,**
**dass** die Tragstange (125) zumindest bereichsweise gabelförmig ausgebildet ist und gegen eine Rückstellkraft zusammendrückbare Gabelarme (137, 139) umfasst, die insbesondere auf ihren einander abgewandten Außenseiten mit Profilierungen (133, 135) versehen sind.

38. Distraktionsvorrichtung nach Anspruch 37,
**dadurch gekennzeichnet,**
**dass** die Gabelarme (137, 139) der Tragstange (125) insbesondere an in entgegengesetzte Richtungen weisenden Seiten mit Profilierungen in Form von unterschiedlich orientierten Rippenstrukturen (133, 135) versehen sind.

39. Distraktionsvorrichtung nach einem der Ansprüche 34 bis 38,
**dadurch gekennzeichnet,**
**dass** die Tragstange (125) insbesondere zur Höhenverstellung der Schnittlehre zumindest längs der Distraktionsachse relativ zum Tragkörper (43) verstellbar ist.

40. Distraktionsvorrichtung nach einem der Ansprüche 34 bis 39,
**dadurch gekennzeichnet,**
**dass** die Tragstange (125) und der Tragkörper (43) zumindest bei Verstellbewegungen längs der Distraktionsachse nach Art einer Ratsche zusammenwirken.

41. Distraktionsvorrichtung nach einem der Ansprüche 34 bis 40,
**dadurch gekennzeichnet,**
**dass** die Tragstange (125) aus einer parallelen Ausrichtung zur Distraktionsachse heraus gegenüber dem Tragkörper (43) verschwenkbar ist, insbesondere in einer von zwei zu beiden Seiten des Verstellkanals (127) angeordneten Kolben-/Zylinder-Anordnungen festgelegten Ebene.

42. Distraktionsvorrichtung nach Anspruch 41,
**dadurch gekennzeichnet,**
**dass** die Tragstange (125) einen auf die Breite des Verstellkanals (127) abgestimmten Schwenkkopf (157) aufweist, um welchen die Tragstange (125) verschwenkbar ist.

43. Distraktionsvorrichtung nach einem der Ansprüche 34 bis 42,
**dadurch gekennzeichnet,**
**dass** sich der Verstellkanal (127) in bei eingesteckter Tragstange (125) von der Plattform (123) weg weisender Richtung erweitert.

44. Distraktionsvorrichtung nach einem der Ansprüche 34 bis 43,
**dadurch gekennzeichnet,**
**dass** die Tragstange (125) mit einer Anzeigeeinrichtung (141), insbesondere in Form einer Skala, versehen ist, die im in den Verstellkanal (127) eingeführten Zustand über ein in einer Wand des Tragkörpers (43) ausgebildetes Ablesefenster (143) von außen sichtbar ist.

45. Distraktionsvorrichtung nach einem der Ansprüche 34 bis 44,
**dadurch gekennzeichnet,**
**dass** die Plattform (123) bei zum Aufspreizen des Knies zwischen Femur und Tibia eingebrachten Arbeitsabschnitten (11, 13) zumindest im Wesentlichen außerhalb der Projektion der Tibia längs der Distraktionsachse gelegen ist.

46. Distraktionsvorrichtung nach einem der Ansprüche 34 bis 45,
**dadurch gekennzeichnet,**
**dass** die Plattform (123) vor einem Funktionsblock (47) gelegen ist, der sich parallel zur Distraktionsachse erstreckt und mit dem der mit dem Zylinder koppelbare Arbeitsabschnitt (13) fest verbunden ist.

47. Distraktionsvorrichtung nach einem der Ansprüche 32 bis 46,
**dadurch gekennzeichnet,**
**dass** der obere Arbeitsabschnitt (11) mit einer Anzeigeeinrichtung (145), insbesondere in Form einer Skala, versehen ist.

## Claims

1. A hydraulic traction apparatus for surgery, in particular for knee surgery, having at least one pair of working sections (11, 13) which can be adjusted relative to one another and at least one piston in cylinder arrangement (15, 17) whose piston (15) is releasably coupled to the one working section (11) and whose cylinder (17) is coupled to the other working section (13),
**characterized**
**in that** the piston in cylinder arrangement (15, 17) is replaceable and is made of plastic, with the coupling of the piston (15) and of the cylinder (17) to the working sections (11, 13) being releasable in each case; and
**in that** the piston in cylinder arrangement (15, 17) is stiffened by an outer support mount (19) supporting the cylinder (17) from the outside and by at least one inner reinforcement insert (21, 23, 25) which can be introduced into the cylinder (17) together with the piston (15).

2. A traction apparatus in accordance with claim 1, **characterized in that** the piston (15) and the cylinder are each disposable articles produced by the injection molding method.

3. A traction apparatus in accordance with claim 1 or claim 2, **characterized in that** the piston (15) and the reinforcement insert (21, 23, 25) form a compact piston unit in the assembled state which completely fills the interior of the cylinder (17) at least over a partial peripheral region.

4. A traction apparatus in accordance with any one of the preceding claims, **characterized in that** the piston in cylinder arrangement (15, 17) is formed by a commercial disposable syringe.

5. A traction apparatus in accordance with any one of the preceding claims, **characterized in that** a free space present between the inner wall of the cylinder (17) and a piston rod (27) is at least partly filled by the reinforcement insert (21, 23).

6. A traction apparatus in accordance with any one of the preceding claims, **characterized in that** at least one chamber (29), which is present in the cylinder (17) and which is bounded by a rib structure (31) of a piston rod (27), which extends up to the inner wall of the cylinder (17) and is in particular cross-shaped or X-shaped in cross-section, is at least partly filled by the reinforcement insert (21, 23).

7. A traction apparatus in accordance with claim 6, **characterized in that** at least one pair of chambers disposed opposite one another is each at least partly filled by a reinforcement insert (21, 23).

8. A traction apparatus in accordance with any one of the claims 1 to 5, **characterized in that** the reinforcement insert (25) completely fills the interior of a tubular piston rod (27) guided at the inner wall of the cylinder (17) at least over a partial peripheral region.

9. A traction apparatus in accordance with any one of the preceding claims, **characterized in that** the piston in cylinder arrangement (15, 17) can be coupled to the working sections (11, 13) in the assembled state.

10. A traction apparatus in accordance with any one of the preceding claims, **characterized in that** at least one reinforcement insert (21, 25) is fixedly connected to the one working section (11).

11. A traction apparatus in accordance with any one of the preceding claims, **characterized in that** at least one reinforcement insert (23) is provided in the form of a separate component.

12. A traction apparatus in accordance with any one of the preceding claims, **characterized in that** the support mount (19) for the cylinder (17) and the working section (13), which can be coupled to the cylinder (17), are fixedly connected to a carrier member (43).

13. A traction apparatus in accordance with any one of the preceding claims, **characterized in that** a handle (45), which is preferably fixedly connected to a carrier member (43), is provided at the opposite side of the piston in cylinder arrangement (15, 17) from the working sections (11, 13).

14. A traction apparatus in accordance with any one of the preceding claims, **characterized in that** the working section (13), which can be coupled to the cylinder (17), is fixedly connected to a functional block (47) which extends parallel to the traction direction and which is formed as a security against rotation and/or deflection for the working section (11) which can be coupled to the piston (15) .

15. A traction apparatus in accordance with claim 14, **characterized in that** the functional block (47) has an outer cross-section deviating from a circular shape and is engaged over, at least over a partial peripheral region, by a guide section (49) of the work section (11) in a manner secured against rotation.

16. A traction apparatus in accordance with claim 14 or claim 15, **characterized in that** the functional block (47) has an abutment surface (51) which is slightly inclined with respect to the traction direction, runs toward the longitudinal axis of the piston in cylinder arrangement (15, 17) in the direction of increasing traction length and cooperates with the working section (11) in a manner securing against deflection.

17. A traction apparatus in accordance with any one of the claims 14 to 16, **characterized in that** the functional block (47) is provided with a display device (53), in particular in the form of a scale attached to an outer side, at which a measure for the traction length can be read off.

18. A traction apparatus in accordance with any one of the preceding claims, **characterized in that** the support mount (19) and/or the reinforcement insert (21, 23, 25) are produced from a material with a higher bending strength than the plastic of the piston in cylinder arrangement (15, 17), in particular from a metal or from a fiber reinforced plastic.

19. A traction apparatus in accordance with any one of the preceding claims, **characterized in that** the piston in cylinder arrangement (15, 17) is provided with a display device, in particular in the form of a scale attached to the cylinder.

20. A traction apparatus in accordance with any one of the preceding claims, **characterized in that** a fluid supply device (33) for the piston in cylinder arrangement (15, 17) includes a syringe (35) operable by hand, in particular a disposable syringe.

21. A traction apparatus in accordance with any one of the preceding claims, **characterized in that** at least one pressure display device (39) is connected to a fluid line (37) connecting the piston in cylinder arrangement (15, 17) to a fluid supply device (33).

22. A traction apparatus in accordance with any one of the preceding claims, **characterized in that** the piston in cylinder arrangement (15, 17) is a component of a hydraulic system which can be handled as a unit, which can in particular be sterilized and which additionally includes at least one fluid supply device (33), a fluid line (37) connecting the piston in cylinder arrangement (15, 17) to the fluid supply device (33) as well as a hydraulic fluid, in particular water, and can be coupled to the working sections (11, 13) as a whole.

23. A traction apparatus in accordance with any one of the preceding claims, **characterized in that** at least two piston in cylinder arrangements (15, 17) are provided which are active in parallel directions in the state coupled to the working sections (11, 13).

24. A traction apparatus in accordance with claim 23, **characterized in that** the piston in cylinder arrangements (15, 17) are connected to a common fluid supply device (33) during use.

25. A traction apparatus in accordance with claim 23 or claim 24, **characterized in that** the piston in cylinder arrangements (15, 17) can be selectively connected to the fluid supply or be separated from the fluid supply independently of one another by on/off valves (41) arranged in a fluid line (37) connecting the piston in cylinder arrangements (15, 17) to a fluid supply device (33).

26. A traction apparatus in accordance with any one of the preceding claims, **characterized in that** the working sections (11, 13) form a spreading section which can be introduced between the femur (61) and the tibia (63) and which extends substantially perpendicular to the traction direction for the setting of desired spacings between the femur (61) and the tibia (63).

27. A traction apparatus in accordance with any one of the preceding claims, **characterized in that** an upper working section (11) is provided with a support section and/or fastening section (67, 81), via which the apparatus can be supported and/or fastened to the tibia (63), for the determination of the ligament tension and/or of the ligament length on the implanting of anterior cruciate ligaments (65).

28. A traction apparatus in accordance with claim 27, **characterized in that** the working sections (11, 13) are each provided with at least one cut-out (69) for the passage of a fixing tool (71) and of a fixing member for the fixing of the ligament (65) in the tibia (63).

29. A traction apparatus in accordance with claim 27 or claim 28, **characterized in that** the upper working section (11) is provided with at least one cut-out (69) for the passage of the ligament (65) and/or of a drawing element (73) connected to the ligament (65).

30. A traction apparatus in accordance with any one of the claims 27 to 29, **characterized in that** a lower section (13) is provided with at least one fastening section (30) in order to fasten the ligament (65) or a drawing element (73) connected to the ligament (65).

31. A traction apparatus in accordance with any one of the preceding claims, **characterized in that** the working sections (11, 13) are each formed as tongue-like platforms which extend at least substantially parallel to one another in the assembled state.

32. A traction apparatus in accordance with any one of the preceding claims, **characterized in that** the support mount (19) for the cylinder is made at least partly tubular and the cylinder is insertable into the support mount (19) along the tube axis, with the support mount (19) having an insertion opening (121), in particular a slot-shaped insertion opening, for the lateral insertion of a fluid line connected to the cylinder.

33. A traction apparatus in accordance with any one of the preceding claims, **characterized in that** a carrier (123, 125) is provided for a cutting gauge, in particular for a cutting block, which is couplable to a carrier element (43) to which the support mount (19) for the cylinder and the working section (13) couplable to the cylinder are connected.

34. A traction apparatus in accordance with claim 33, **characterized in that** the carrier includes a platform (123) serving as a support for the cutting gauge and a carrier bar (125) which can be coupled to the carrier element (43) and which carries the platform (123) at its one end and is insertable into an adjustment passage (127) of the carrier element (43) in which the carrier bar (125) is adjustable relative to the carrier element (43) and is fixable in a plurality of positions, in particular discretely distributed positions, on the carrier element (43).

35. A traction apparatus in accordance with claim 34, **characterized in that** the carrier bar (125) and the inner wall of the adjustment passage (127) are provided with structures (129, 131, 133, 135), in particular rib-shaped structures, which cooperate for the fixing of their relative position.

36. A traction apparatus in accordance with claim 34 or claim 35, **characterized in that** the adjustment arrangement is made in a self-locking manner from the carrier bar (125) and the adjustment passage (127).

37. A traction apparatus in accordance with any one of the claims 34 to 36, **characterized in that** the carrier bar (125) is made in fork shape at least regionally and includes fork arms (137, 139) which can be pressed together against a restoring force and which are in particular provided with structures (133, 135) on their outer sides facing away from one another.

38. A traction apparatus in accordance with claim 37, **characterized in that** the fork arms (137, 139) of the carrier bar (125) are provided with structures in the form of differently oriented rib structures (133, 135) in particular at sides facing in opposite directions.

39. A traction apparatus in accordance with any one of the claims 34 to 38, **characterized in that** the carrier bar (125) is adjustable in particular for the vertical adjustment of the cutting gauge relative to the carrier element (43) at least along the traction axis.

40. A traction apparatus in accordance with any one of claims 34 to 39, **characterized in that** the carrier bar (125) and the carrier element (43) cooperate in the manner of a ratchet at least on adjustment movements along the traction axis.

41. A traction apparatus in accordance with any one of the claims 34 to 40, **characterized in that** the carrier bar (125) can be pivoted out of a parallel alignment to the traction axis with respect to the carrier element (43), in particular in a plane fixed by two piston-in-cylinder arrangements arranged at both sides of the adjustment passage (127).

42. A traction apparatus in accordance with claim 41, **characterized in that** the carrier bar (125) has a pivot head (157) which is matched to the width of the adjustment passage (127) and about which the carrier bar (125) is pivotable.

43. A traction apparatus in accordance with any one of the claims 34 to 42, **characterized in that** the adjustment passage (127) widens in the direction pointing away from the platform (123) when the carrier bar (125) is inserted.

44. A traction apparatus in accordance with any one of the claims 34 to 43, **characterized in that** the carrier bar (125) is provided with a display device (141), in particular in the form of a scale, which is visible from the outside via a reading window (143) formed in a wall of the carrier element (43) in the state introduced into the adjustment passage (127).

45. A traction apparatus in accordance with any one of the claims 34 to 44, **characterized in that** the platform (123) is disposed at least substantially outside the projection of the tibia along the traction axis when the working sections (11, 13) are introduced between the femur and the tibia for the spreading apart of the knee.

46. A traction apparatus in accordance with any one of the claims 34 to 45, **characterized in that** the platform (123) is disposed in front of a functional block (47) which extends parallel to the traction axis and to which the working section (13) couplable to the cylinder is firmly connected.

47. A traction apparatus in accordance with any one of the claims 32 to 46, **characterized in that** the upper working section (11) is provided with a display device (145), in particular in the form of a scale.

## Revendications

1. Dispositif d'écartement hydraulique pour opération, en particulier pour des opérations du genou, comprenant au moins une paire de tronçons de travail (11, 13) déplaçables l'un par rapport à l'autre, et au moins un agencement à piston-et-cylindre (15, 17) dont le piston (15) est couplé à l'un des tronçons de travail (11) et dont le cylindre (17) est couplé à l'autre tronçon de travail (13),
**caractérisé en ce que**
l'agencement à piston-et-cylindre (15, 17) est réalisé de façon interchangeable et en matière plastique, l'accouplement du piston (15) et du cylindre (17) avec les tronçons de travail (11, 13) étant à chaque fois détachable, et
**en ce que** l'agencement à piston-et-cylindre (15, 16) est rigidifié par un dispositif de réception et de soutien (19) extérieur qui soutient le cylindre (17) depuis l'extérieur, et par au moins un insert de renforcement intérieur (21, 23, 25) susceptible d'être introduit conjointement avec le piston (15) dans le cylindre (17).

2. Dispositif d'écartement selon la revendication 1,
**caractérisé en ce que** le piston (15) et le cylindre sont chacun des articles à jeter produits par un procédé de moulage par injection.

3. Dispositif d'écartement selon la revendication 1 ou 2,
**caractérisé en ce que** le piston (15) et l'insert de renforcement (21, 23, 25) forment, à l'état assemblé, une unité de piston compacte qui remplit totalement l'intérieur du cylindre (17) au moins sur une zone périphérique partielle.

4. Dispositif d'écartement selon l'une des revendications précédentes,
**caractérisé en ce que** l'agencement à piston-et-cylindre (15, 17) est formé par une seringue à jeter du commerce.

5. Dispositif d'écartement selon l'une des revendications précédentes,
**caractérisé en ce qu'**un espace libre présent entre la paroi intérieure du cylindre (17) et une tige de piston (27) est rempli au moins partiellement par l'insert de renforcement (21, 23).

6. Dispositif d'écartement selon l'une des revendications précédentes,
**caractérisé en ce qu'**au moins une chambre (29) présente dans le cylindre (17), qui est délimitée par une structure à nervures (31) d'une tige de piston (27), en particulier à section en forme de croix ou en forme de X, qui s'étend jusqu'à la paroi intérieure de cylindre (17), est remplie au moins partiellement par l'insert de renforcement (21, 23).

7. Dispositif d'écartement selon la revendication 6,
**caractérisé en ce qu'**au moins une paire de chambres mutuellement opposées sont remplies au moins partiellement par un insert de renforcement (21, 23) respectif.

8. Dispositif d'écartement selon l'une des revendications 1 à 5,
**caractérisé en ce que** l'insert de renforcement (25) remplit totalement et au moins sur une zone périphérique partielle l'intérieur d'une tige de piston tubulaire (27) guidée contre la paroi intérieure de cylindre (17).

9. Dispositif d'écartement selon l'une des revendications précédentes,
**caractérisé en ce que** l'agencement à piston-et-cylindre (15, 17) est susceptible d'être accouplé, à l'état assemblé, avec les tronçons de travail (11, 13).

10. Dispositif d'écartement selon l'une des revendications précédentes,
**caractérisé en ce qu'**au moins un insert de renforcement (21, 25) est relié solidairement à l'un des tronçons de travail (11).

11. Dispositif d'écartement selon l'une des revendications précédentes,
**caractérisé en ce qu'**au moins un insert de renforcement (23) est prévu sous la forme d'un composant séparé.

12. Dispositif d'écartement selon l'une des revendications précédentes,
**caractérisé en ce que** le dispositif de réception et de soutien (19) pour le cylindre (17) et le tronçon de travail (13) à accoupler avec le cylindre. (17) sont reliés solidairement avec un corps porteur (43).

13. Dispositif d'écartement selon l'une des revendications précédentes,
**caractérisé en ce que**, sur le côté, opposé aux tronçons de travail (11, 13), de l'agencement à piston-et-cylindre (15, 17) est prévue une poignée (45) qui est de préférence reliée solidairement avec un corps porteur (43).

14. Dispositif d'écartement selon l'une des revendications précédentes,
**caractérisé en ce que** le tronçon de travail (13) à accoupler avec le cylindre (17) est relié solidairement à un bloc fonctionnel (47) qui s'étend parallèlement à la direction d'écartement et qui est réalisé à titre de sécurité antirotation et/ou de sécurité anti-déviation pour le tronçon de travail (11) à accoupler avec le piston (15).

15. Dispositif d'écartement selon la revendication 14,
**caractérisé en ce que** le bloc fonctionnel (47) présente une section extérieure qui diffère d'une forme circulaire et est entouré au moins sur une zone périphérique partielle par un tronçon de guidage (49) du tronçon de travail (11) en assurant une sécurité antirotation.

16. Dispositif d'écartement selon la revendication 14 ou 15,
**caractérisé en ce que** le bloc fonctionnel (47) présente une surface de butée (51) légèrement inclinée par rapport à la direction d'écartement et convergeant vers l'axe longitudinal de l'agencement à piston-et-cylindre (15, 17) dans la direction dans laquelle la longueur d'écartement augmente, surface de butée qui coopère avec le tronçon de travail (11) pour assurer une sécurité anti-déviation.

17. Dispositif d'écartement selon l'une des revendications 14 à 16,
**caractérisé en ce que** le bloc fonctionnel (47) est pourvu d'un moyen d'affichage (53), en particulier sous la forme d'une échelle appliquée sur une face extérieure, sur laquelle une mesure pour la longueur d'écartement peut être lue.

18. Dispositif d'écartement selon l'une des revendications précédentes,
**caractérisé en ce que** le dispositif de réception et de soutien (19) et/ou l'insert de renforcement (21, 23, 25) sont réalisés en un matériau qui présente une résistance à la flexion plus élevée que la matière plastique de l'agencement à piston-et-cylindre (15, 17), en particulier en métal ou en matière plastique renforcée de fibres.

19. Dispositif d'écartement selon l'une des revendications précédentes,
**caractérisé en ce que** l'agencement à piston-et-cylindre (15, 17) est pourvu d'un moyen d'affichage, en particulier sous la forme d'une échelle appliquée sur le cylindre.

20. Dispositif d'écartement selon l'une des revendications précédentes,
**caractérisé en ce qu'**un dispositif d'amenée de fluide (33) pour l'agencement à piston-et-cylindre (15, 17) comprend une seringue (35) actionnée à la main, en particulier une seringue à jeter.

21. Dispositif d'écartement selon l'une des revendications précédentes,
**caractérisé en ce qu'**un dispositif indicateur de pression est raccordé à une conduite à fluide (37) qui relie l'agencement à piston-et-cylindre (15, 17) avec un dispositif d'amenée de fluide (33).

22. Dispositif d'écartement selon l'une des revendications précédentes,
**caractérisé en ce que** l'agencement à piston-et-cylindre (15, 17) fait partie d'un système hydraulique fermé qui peut être manipulé comme une unité et en particulier stérilisable, qui comprend additionnellement au moins un dispositif d'amenée de fluide (33), une conduite à fluide (37) qui relie l'agencement à piston-et-cylindre (15,17) avec le dispositif d'amenée de fluide (33), et un fluide hydraulique, en particulier de l'eau, et est susceptible d'être accouplé sous forme d'un tout avec les tronçons de travail (11, 13).

23. Dispositif d'écartement selon l'une des revendications précédentes,
**caractérisé en ce qu'**il est prévu au moins deux agencements à piston-et-cylindre (15, 17) qui, dans l'état accouplé avec les tronçons de travail (11, 13), agissent dans des directions parallèles.

24. Dispositif d'écartement selon la revendication 23,
**caractérisé en ce que** les agencements à piston-et-cylindre (15, 17) sont raccordés, pendant l'utilisation, à un dispositif d'amenée de fluide (33) commun.

25. Dispositif d'écartement selon la revendication 23 ou 24,
**caractérisé en ce que**, au moyen de vannes de commutation (41) agencées dans une conduite à fluide (37) qui relie les agencements à piston-et-cylindre (15, 17) avec un dispositif d'amenée de fluide (33), les agencements à piston-et-cylindre (15,17) sont susceptibles d'être reliés sélectivement et indépendamment l'un de l'autre avec l'amenée de fluide ou séparés de l'amenée de fluide.

26. Dispositif d'écartement selon l'une des revendications précédentes,
**caractérisé en ce que**, pour établir des distances de consigne entre le fémur (61) et le tibia (63), les tronçons de travail (11, 13) forment une unité d'élargissement susceptible d'être introduite entre le fémur (61) et le tibia (63) et s'étendant essentiellement perpendiculairement à la direction d'écartement.

27. Dispositif d'écartement selon l'une des revendications précédentes,
**caractérisé en ce que**, pour déterminer la tension et/ou la longueur des ligaments lors de l'implantation de ligaments croisés antérieurs (65), un tronçon de travail supérieur (11) est pourvu d'un tronçon de soutien et/ou de fixation (67, 81), au moyen duquel le dispositif peut être soutenu et/ou fixé sur le tibia (63).

28. Dispositif d'écartement selon la revendication 27,
**caractérisé en ce que** les tronçons de travail (11, 13) sont chacun pourvus d'au moins un évidement (69) pour la traversée d'un outil de fixation (71) et d'un élément de fixation pour fixer le ligament (65) dans le tibia (63).

29. Dispositif d'écartement selon la revendication 27 ou 28,
**caractérisé en ce que** le tronçon de travail supérieur (11) est pourvu d'au moins un évidement (69) pour la traversée du ligament (65) et/ou d'un élément de traction (73) relié au ligament (65).

30. Dispositif d'écartement selon l'une des revendications 27 à 29,
**caractérisé en ce qu'**un tronçon de travail inférieur (13) est pourvu d'au moins un tronçon de fixation pour fixer le ligament (65) ou un élément de traction (73) relié au ligament (65).

31. Dispositif d'écartement selon l'une des revendications précédentes,
**caractérisé en ce que** les tronçons de travail (11, 13) sont réalisés chacun comme des plates-formes semblables à des languettes qui, dans l'état assemblé, s'étendent au moins sensiblement parallèlement l'une à l'autre.

32. Dispositif d'écartement selon l'une des revendications précédentes,
**caractérisé en ce que** le dispositif de réception et de soutien (19) pour le cylindre est réalisé au moins sous forme tubulaire partielle, et le cylindre est susceptible d'être introduit dans le dispositif de réception et de soutien (19) le long de l'axe du tube, et ledit dispositif de réception et de soutien (19) présente, pour l'introduction latérale d'une conduite à fluide raccordée au cylindre, une ouverture de mise en place (121) en particulier en forme de fente.

33. Dispositif d'écartement selon l'une des revendications précédentes,
**caractérisé en ce qu'**il est prévu un support (123, 125) pour un gabarit de coupe, en particulier un bloc de coupe qui est susceptible d'être couplé à un corps porteur (43) auquel sont reliés le dispositif de réception et de soutien (19) pour le cylindre et le tronçon de travail (13) susceptible d'être accouplé au cylindre.

34. Dispositif d'écartement selon la revendication 33,
**caractérisé en ce que** le support comprend une plate-forme (123) servant d'appui pour le gabarit de coupe et une tige de traction (125) susceptible d'être accouplée au corps porteur (43), qui porte à l'une de ses extrémités la plate-forme (123) et qui peut être introduite dans un canal de déplacement (127) du corps porteur (43), dans lequel la tige portante (125) est déplaçable par rapport au corps porteur (43) est peut être fixée sur le corps porteur (43) dans une pluralité de positions, en particulier distribuées de manière discrète.

35. Dispositif d'écartement selon la revendication 34,
**caractérisé en ce que** la tige portante (125) et la paroi intérieure du canal de déplacement (127) sont dotées de profilages (129, 131, 133, 135), en particulier en forme de nervures, qui coopèrent pour fixer leurs positions relatives.

36. Dispositif d'écartement selon la revendication 34 ou 35,
**caractérisé en ce que** l'agencement déplaçable formé par la tige portante (125) et le canal de déplacement (127) est réalisé avec auto-coincement.

37. Dispositif d'écartement selon l'une des revendications 34 à 36,
**caractérisé en ce que** la tige portante (125) est réalisée au moins localement en forme de fourche et comprend des bras de fourche (137, 139) susceptibles d'être poussés l'un vers l'autre à l'encontre d'une force de rappel, qui sont dotés de profilages (133, 135), en particulier sur leurs faces extérieures détournées l'une de l'autre.

38. Dispositif d'écartement selon la revendication 37,
**caractérisé en ce que** les bras de fourche (137, 139) de la tige portante (125) sont pourvus, en particulier sur des faces tournées dans des directions opposées, de profilages sous la forme de structures en nervures (133, 135) avec des orientations différentes.

39. Dispositif d'écartement selon l'une des revendications 34 à 38,
**caractérisé en ce que** la tige portante (125) est déplaçable au moins le long de l'axe d'écartement par rapport au corps porteur (43), en particulier pour le réglage en hauteur du gabarit de coupe.

40. Dispositif d'écartement selon l'une des revendications 34 à 39,
**caractérisé en ce que** la tige portante (125) et le corps porteur (43) coopèrent à la manière d'un cliquet, au moins lors de déplacements de réglage le long de l'axe d'écartement.

41. Dispositif d'écartement selon l'une des revendications 34 à 40,
**caractérisé en ce que** la tige portante (125) est capable de pivoter depuis une orientation parallèle à l'axe d'écartement par rapport au corps porteur (43), en particulier dans un plan défini par deux agencements à piston-et-cylindre agencés des deux côtés du canal de déplacement (127).

42. Dispositif d'écartement selon la revendication 41,
**caractérisé en ce que** la tige portante (125) comprend une tête de pivotement (157) adaptée à la largeur du canal de déplacement (127), autour de laquelle la tige portante (125) est capable de pivoter.

43. Dispositif d'écartement selon l'une des revendications 34 à 42,
**caractérisé en ce que** le canal de déplacement (127) s'élargit dans une direction en éloignement de la plate-forme (123) lorsque la tige portante (125) est enfichée.

44. Dispositif d'écartement selon l'une des revendications 34 à 43;
**caractérisé en ce que** la tige portante (125) est pourvue d'un dispositif d'affichage (141), en particulier sous la forme d'une échelle qui, dans l'état introduit dans le canal de déplacement (127); est visible de l'extérieur via une fenêtre de lecture (143) réalisée dans une paroi du corps porteur (43).

45. Dispositif d'écartement selon l'une des revendications 34 à 44,
**caractérisé en ce que** la plate-forme (123), lorsque les tronçons de travail (11, 13) sont introduits entre le fémur et le tibia pour assurer l'ouverture du genou, est disposée au moins sensiblement à l'extérieur de la projection du tibia le long de l'axe d'écartement.

46. Dispositif d'écartement selon l'une des revendications 34 à 45,
**caractérisé en ce que** la plate-forme (123) est placée devant un bloc fonctionnel (47) qui s'étend parallèlement à l'axe d'écartement et qui est relié solidairement au tronçon de travail (13) à accoupler au cylindre.

47. Dispositif d'écartement selon l'une des revendications 32 à 46,
**caractérisé en ce que** le tronçon de travail supérieur (11) est pourvu d'un dispositif d'affichage (145), en particulier sous la forme d'une échelle.
